(19) Europäisches Patentamt
European Patent Office
Office européen des brevets

(11) **EP 3 376 286 A1**

(12) **EUROPEAN PATENT APPLICATION**
published in accordance with Art. 153(4) EPC

(43) Date of publication:
**19.09.2018 Bulletin 2018/38**

(51) Int Cl.:
*G03F 7/038* (2006.01)    *G01N 37/00* (2006.01)

(21) Application number: **16864268.4**

(22) Date of filing: **09.11.2016**

(86) International application number:
**PCT/JP2016/083268**

(87) International publication number:
**WO 2017/082307 (18.05.2017 Gazette 2017/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **10.11.2015 JP 2015220176**
**08.07.2016 JP 2016136415**

(71) Applicant: **Asahi Glass Company, Limited**
**Tokyo 100-8405 (JP)**

(72) Inventors:
• **SAKURADA, Tomoaki**
 **Tokyo 100-8405 (JP)**
• **OBI, Masaki**
 **Tokyo 100-8405 (JP)**

(74) Representative: **Müller-Boré & Partner**
**Patentanwälte PartG mbB**
**Friedenheimer Brücke 21**
**80639 München (DE)**

(54) **PHOTOSENSITIVE COMPOSITION FOR BIOCHIP FOR FLUORESCENCE ANALYSIS, METHOD FOR MANUFACTURING BIOCHIP FOR FLUORESCENCE ANALYSIS, AND BIOCHIP FOR FLUORESCENCE ANALYSIS**

(57)    To provide a biochip whereby noise lights in fluorescence analysis are reduced. A photosensitive composition for forming a liquid repellent film on a liquid contact surface of a biochip for fluorescence analysis, said photosensitive resin composition comprising a polymer having a fluoroalkyl group which may have an etheric oxygen atom, and a polymerizable crosslinkable group, and a photoinitiator having an absorption coefficient at a wavelength of 365 nm of at most 400 [mL·g$^{-1}$·cm$^{-1}$]. A method for producing a biochip for fluorescence analysis, which comprises applying the photosensitive composition onto a liquid contact surface of a biochip for fluorescence analysis, followed by exposure and development.

EP 3 376 286 A1

**Description**

TECHNICAL FIELD

The present invention relates to a photosensitive composition to be used for forming a liquid-repellent film on a liquid contact surface of a biochip for fluorescence analysis, a method for producing a biochip for fluorescence analysis, and a biochip for fluorescence analysis.

BACKGROUND ART

A biochip is a device which is capable of detecting, in mass and concurrently, biomolecules and target compounds such as DNA, proteins, sugar chains, etc. As a highly sensitive detection method, it has been in practice that using beads, etc. labeled with another antibody, etc. interacting specifically with a target molecule, after the target molecule and beads are allowed to interact, the beads are sealed in microarrays in a biochip, followed by detection. In such detection, fluorescence analysis is used in many cases.

Further, in such a biochip, in order to let many beads be efficiently sealed in arrays, housing side walls and upper surfaces of the arrays to accommodate the beads are provided with water repellency, and housing bottom surfaces are provided with hydrophilicity. For that purpose, it has been in practice that a water-repellent layer is provided on a hydrophilic substrate, and holes are formed in the water-repellent layer by lithography to let hydrophilic portions and water-repellent portions be separately formed (e.g. see Patent Document 1).

Further, in the production of another example of a biochip (to fix biomolecules to the chip), at the time of fixing biomolecules onto the substrate, it is necessary to determine the fixing positions exactly. For that purpose, it has been in practice that a water-repellent layer is provided on a hydrophilic substrate, and holes are formed in the water-repellent layer by lithography to let hydrophilic portions and water-repellent portions be separately formed (e.g. see Patent Document 2).

This water-repellent layer is desired to be one having good adhesion to the substrate, capable of being processed by lithography, having good water repellency, and having light causing noise at the time of fluorescence analysis been suppressed (e.g. see Patent Document 3).

PRIOR ART DOCUMENTS

PATENT DOCUMENTS

Patent Document 1: WO 2012/121310
Patent Document 2: WO 2006/129800
Patent Document 3: JP-A-2009-075261

DISCLOSURE OF INVENTION

TECHNICAL PROBLEM

The present invention has an object to provide a biochip which is excellent in liquid repellency and whereby light which becomes noise at the time of fluorescence analysis is suppressed.

SOLUTION TO PROBLEM

The present invention is an invention shown in the following.

[1] A photosensitive composition which comprises the following polymer (A), or the following polymer (B) and the following polymer (C), and further a photoinitiator having an absorption coefficient at a wavelength of 365 nm of at most 400 [mL·g$^{-1}$·cm$^{-1}$], and which is to be used to form a liquid-repellent film on a liquid contact surface of a biochip for fluorescence analysis,

Polymer (A): a polymer having a polymerizable crosslinkable group and a fluoroalkyl group which may have an etheric oxygen atom between carbon atoms,

Polymer (B): a polymer other than the polymer (A), having a fluoroalkyl group which may have an etheric oxygen atom between carbon atoms,

Polymer (C): a polymer other than the polymer (A), having a polymerizable crosslinkable group.

[2] The photosensitive composition according to [1], wherein the fluoroalkyl group in the polymer (A) and the polymer (B) is a fluoroalkyl group having a perfluoroalkyl moiety having 4, 5 or 6 carbon atoms.

[3] The photosensitive composition according to [1], wherein the fluoroalkyl group in the polymer (A) and the polymer (B) is a fluoroalkyl group having a perfluoroalkyl moiety having 4 to 8 carbon atoms and having from 1 to 3 etheric oxygen atoms between carbon atoms.

[4] The photosensitive composition according to any one of [1] to [3], wherein the polymerizable crosslinkable group in the polymer (A) and the polymer (C) is a group having an ethylenic double bond, a group having a three-membered cyclic ether structure, or a group having a four-membered cyclic ether structure.

[5] The photosensitive composition according to any one of [1] to [4], wherein the weight average molecular weight of each of the polymer (A), the polymer (B) and the polymer (C) is from 5,000 to 500,000.

[6] The photosensitive composition according to any one of [1] to [5], wherein the photosensitive composition is a photosensitive composition comprising the polymer (A).

[7] The photosensitive composition according to [6], wherein the polymer (A) is a polymer comprising a structural unit having a fluoroalkyl group which may have an etheric oxygen atom between carbon atoms, a structural unit having a polymerizable crosslinkable group, and optionally a structural unit other than the above units.

[8] The photosensitive composition according to [7], wherein the content proportions of the respective structural units in the polymer (A) are such that, based on all structural units, the structural unit having a fluoroalkyl group is from 20 to 95 mol%, the structural unit having a polymerizable crosslinkable group is from 5 to 80 mol%, and the structural unit other than the above units is from 0 to 60 mol%.

[9] The photosensitive composition according to any one of [1] to [8], wherein the photoinitiator is a photo-radical generator or a photo-acid generator.

[10] The photosensitive composition according to any one of [1] to [9], wherein the photosensitive composition further contains a solvent.

[11] A method for producing a biochip for fluorescence analysis, characterized by applying the photosensitive composition as defined in any one of [1] to [10] on a liquid contact surface of a substrate of the biochip for fluorescence analysis, and, when the coating film of the photosensitive composition has a solvent, removing said solvent, followed by exposure and development, to form a liquid-repellent film having through-holes formed in the thickness direction.

[12] The method for producing a biochip for fluorescence analysis according to [11], wherein the substrate is a substrate having a lyophilic surface, and the liquid-repellent film is formed on the lyophilic surface of the substrate.

[13] The method for producing a biochip for fluorescence analysis according to [11] or [12], wherein the fluorine content in the liquid-repellent film formed is from 15 to 75 mass%.

[14] A biochip for fluorescence analysis comprising a substrate and a liquid-repellent film provided on a liquid contact surface of the substrate and having through-holes formed in the thickness direction, wherein

the liquid-repellent film has a fluorescence intensity of at most 15,000 as measured by the following measurement method, a water contact angle of at least 100 degrees, and a protein adsorption rate Q of at most 5% as measured by the following measurement method,

(Measurement method for fluorescence intensity)

The above liquid-repellent film is formed on a quartz glass substrate in a thickness of 0.8 $\mu$m, and the fluorescence intensity of the liquid-repellent film is measured by a microarray scanner (manufactured by Molecular Devices, GenePix 4000B), under conditions of an excitation wavelength of 532 nm, a laser power of 100% and a photomultiplier voltage of 1,000 V,

(Measurement method for protein adsorption rate Q)

The protein adsorption rate Q is obtained by the following procedure (1) to (6):

(1) Preparation of wells: In each of 3 wells of a microplate with 24 wells, the above liquid-repellent film is formed on the well surface to cover said well surface.

(2) Preparation of a coloring liquid and a protein solution: As a coloring liquid, one obtained by mixing 50 mL of a peroxidase coloring liquid (3,3',5,5'-tetramethylbenzidine) and 50 mL of 3,3',5,5'-tetramethylbenzidine peroxidase substrate, is used, and, as a protein solution, one obtained by diluting a protein (POD-goat anti mouse IgG, manufactured by Biorad) to 16,000-fold with a phosphate buffer solution (D-PBS, manufactured by Sigma), is used.

(3) Adsorption of protein: In the microplate with 24 wells having the liquid-repellent film formed in the above (1), in each of the wells having the liquid-repellent film formed thereon, 2 mL of the protein solution is dispensed and left to stand at room temperature for 1 hour. As a blank, in a microplate with 96 wells uncoated, in each of 3 wells, 2

μL of the protein solution is dispensed.

(4) Washing of wells: The microplate with 24 wells subjected to adsorption of the protein in the above (3) is washed four times with 4 mL of a phosphate buffer solution (D-PBS, manufactured by Sigma) having a surfactant (Tween20, manufactured by Wako Pure Chemical Industries, Ltd.) incorporated in an amount of 0.05 mass%.

(5) Dispensing of coloring liquid: To each well of the microplate with 24 wells after the washing in the above (4), 2 mL of the above coloring liquid is dispensed, and a coloring reaction is carried out for 7 minutes, whereupon 1 mL of 2N sulfuric acid is added to terminate the coloring reaction. As a blank, to each well of the microplate with 96 wells, 100 μL of the coloring liquid is dispensed, and a coloring reaction is carried out for 7 minutes, whereupon 50 μL of 2N sulfuric acid is added to terminate the coloring reaction.

(6) Measurement of absorbance and calculation of protein adsorption rate Q:

From each well of the microplate with 24 wells, 150 μL of liquid is taken and transferred, respectively, to 3 wells of the microplate with 96 wells, whereupon the absorbance at a wavelength of 450 nm is measured by MTP-810Lab (manufactured by Corona Electric Co., Ltd.). Here, the average value of absorbance of the blank is deemed to be Ao. The absorbance of the liquid transferred from the microplate with 24 wells to the microplate with 96 wells is deemed to be $A_1$, and the protein adsorption rate $Q_1$ is obtained by the following formula. The average value of three protein adsorption rates $Q_1$ is taken as the protein adsorption rate Q.

$$Q_1 = A_1/\{A_0 \times (100/\text{dispense volume of the protein solution of blank})\} \times 100 =$$

$$A_1/\{A_0 \times (100/2\ \mu L)\} \times 100\ [\%]$$

[15] The biochip for fluorescence analysis according to [14], wherein the fluorine content in the liquid-repellent film is from 15 to 75 mass%.

ADVANTAGEOUS EFFECTS OF INVENTION

According to the photosensitive composition and the method for producing a biochip for fluorescence analysis of the present invention, it is possible to obtain a biochip whereby light causing noise at the time of fluorescence analysis is suppressed.

The biochip for fluorescence analysis of the present invention is one whereby light causing noise at the time of fluorescence analysis is suppressed.

DESCRIPTION OF EMBODIMENTS

The photosensitive composition to be used to form a liquid-repellent film on a liquid contact surface of the biochip for fluorescence analysis of the present invention comprises a specific polymer and a photoinitiator. Here, in the case of the composition according to the present invention which contains a volatile component such as a solvent, the volatile component is removed by drying after forming a coating film to form a film of the composition containing no volatile component, and light is irradiated to the film of the composition containing no volatile component. Therefore, when the composition according to the present invention contains a volatile component such as a solvent, the "photosensitive composition" of the present invention means that the composition excluding the volatile component such as a solvent is photosensitive.

One embodiment of the present invention is a photosensitive composition comprising the polymer (A) and a photoinitiator. Another embodiment of the present invention is a photosensitive composition comprising the polymer (B), the polymer (C) and a photoinitiator. The photosensitive composition of the present invention may be a photosensitive composition comprising the polymer (A), the polymer (B), the polymer (C) and a photoinitiator, but either one of the above two embodiments is preferred, and particularly preferred is a photosensitive composition comprising the polymer (A) and a photoinitiator.

The fluoroalkyl group in the polymer (A) and the polymer (B) is an alkyl group having at least one fluorine atom. The fluoroalkyl group may be linear, branched or cyclic. The fluoroalkyl group may have an etheric oxygen atom between its carbon atoms. The number of fluorine atoms to the total number of hydrogen atoms and fluorine atoms in the fluoroalkyl group is preferably at least 50%.

The fluoroalkyl group is preferably a fluoroalkyl group having a perfluoroalkyl moiety (provided that the perfluoroalkyl moiety may have an etheric oxygen atom). The fluoroalkyl group is preferably a fluoroalkyl group having a structure of $-R^1-R^F$. Here, $R^F$ represents a perfluoroalkyl moiety which may have an etheric oxygen atom between carbon atoms, and $R^1$ represents an alkylene moiety having no fluorine atom.

In a case where $R^F$ is a perfluoroalkyl moiety having no etheric oxygen atom, the number of its carbon atoms is preferably 4, 5 or 6, particularly preferably 6. $R^F$ may be linear or branched, but is preferably linear.

In a case where $R^F$ is a perfluoroalkyl moiety having an etheric oxygen atom, the number of etheric oxygen atoms is preferably from 1 to 3. The number of carbon atoms in a perfluoroalkyl moiety on the terminal side of an etheric oxygen atom is preferably 1 to 6, and the number of carbon atoms between etheric oxygen atoms, or between an etheric oxygen atom and $R^1$, is preferably from 1 to 4. The perfluoroalkyl moiety having an etheric oxygen atom, as a whole, may be linear or branched, and its total number of carbon atoms is preferably from 3 to 12, more preferably from 4 to 8.

$R^F$ may specifically be $-(CF_2)_3CF_3$, $-(CF_2)_4CF_3$, $-(CF_2)_5CF_3$, $-CF(CF_3)OCF_2CF_2CF_3$, $-CF_2OCF_2CF_2OCF_3$, $-CF_2OCF_2CF_2OCF_2CF_3$, $-CF_2OCF_2CF_2OCF_2CF_2OCF_3$, etc.

$R^1$ may be linear or branched, but is preferably linear. The number of its carbon atoms is preferably from 1 to 6, more preferably from 2 to 4. As $R^1$, $-CH_2CH_2-$ is particularly preferred.

The polymerizable crosslinkable group in the polymer (A) and the polymer (C), is a group crosslinkable by a radical or an acid. The group crosslinkable by a radical may, for example, be a group containing an ethylenic double bond. The group crosslinkable by an acid may, for example, be a group containing a 3-membered cyclic ether structure or a group containing a 4-membered cyclic ether structure, or a vinyloxy group. The group containing an ethylenic double bond may, for example, be a (meth)acryloyl group, an allyl group, a vinyl group, etc. The group containing a 3-membered cyclic ether structure may be an epoxy group. The group containing a 4-membered cyclic ether structure may be an oxetane group. Here, a "(meth)acryloyl group" is a generic term for an acryloyl group and a methacryloyl group, and the same applies hereinafter.

As the polymerizable crosslinkable group, a group containing an ethylenic double bond, a group containing a 3-membered cyclic ether structure and a group containing a 4-membered cyclic ether structure are preferred, and a (meth)acryloyl group, a vinyloxy group, an epoxy group and an oxetane group are more preferred due to high crosslinking reactivity.

As a method for introducing a polymerizable crosslinkable group into a polymer, there may be (1) a method of preliminarily copolymerizing a monomer having a polymerizable crosslinkable group with another monomer, or (2) a method of copolymerizing a monomer having a specific reactive site with another monomer to obtain a polymer, and then reacting a compound (hereinafter referred to as a compound (b)) having a polymerizable crosslinkable group and a reactive functional group capable of bonding to the specific reactive site.

In a case where the polymerizable crosslinkable group is a group containing an ethylenic double bond, since a monomer having the polymerizable crosslinkable group may also react in the polymerization reaction at the time of producing a polymer, the polymerizable crosslinkable group is introduced into a polymer by the above method (2). In a case where the polymerizable crosslinkable group is a group containing a 3-membered or 4-membered cyclic ether structure, since a monomer having the polymerizable crosslinkable group does not usually react in the polymerization reaction, it is possible to introduce the polymerizable crosslinkable group into a polymer by either method of the methods (1) and (2), but it is preferred to introduce the polymerizable crosslinkable group into a polymer by the method (1) since the production is thereby easy.

The specific reaction site may, for example, be a hydroxy group, a carboxy group, an isocyanate group, an epoxy group, an amino group, etc. The reactive functional group capable of bonding to the specific reactive site may, for example, be, in a case where the specific reactive site is a hydroxy group, an isocyanate group, a carboxy group, a haloacyl group, an epoxy group, etc., and, in a case where the specific reactive site is a carboxy group, a hydroxy group, an isocyanate group, an epoxy group, etc.

The polymer (A) is, from such a viewpoint that a fine hole pattern can be formed and further, it will be excellent in an adhesion inhibitory effect of proteins and cells, preferably a polymer having a structural unit (hereinafter referred to as "structural unit (u1)") having the above-mentioned fluoroalkyl group and a structural unit (hereinafter referred to as "structural unit (u2)") having the above-mentioned polymerizable crosslinkable group, and optionally a structural unit (hereinafter "structural unit (u3)") other than the structural unit (u1) and the structural unit (u2). The polymer (B) is preferably a polymer having the structural unit (u1) and the structural unit (u3) and not having the structural unit (u2). The polymer (C) is preferably a polymer having the structural unit (u2) and the structural unit (u3) and not having the structural unit (u1).

The structural unit (u1) is preferably a structural unit having a fluoroalkyl group having the above-mentioned structure of $-R^1-R^F$. The structural unit (u2) is preferably a structural unit having a group containing an ethylenic double bond, a group containing a three-membered cyclic ether structure or a group containing a 4-membered cyclic ether structure.

The structural unit (u1) is preferably a structural unit derived from a monomer (hereinafter referred to as "monomer (a1)") having the above-mentioned fluoroalkyl group and an ethylenic double bond.

The structural unit (u2) is preferably a structural unit derived from a monomer (hereinafter referred to as "monomer (a2)") having the above-mentioned polymerizable crosslinkable group (but excluding an ethylenic double bond) and an ethylenic double bond, or a structural unit formed by reacting the above mentioned compound (b) to a specific reactive site of a structural unit derived from a monomer (hereinafter referred to as "monomer (a2')") having a specific reactive site and an ethylenic double bond.

The structural unit (u3) is a structural unit derived from a monomer (hereinafter referred to as "monomer (a3)") having an ethylenic double bond other than the above monomer and may further be a structural unit derived from the monomer (a2') and having an unreacted specific reactive site.

The polymer (A) is preferably a polymer obtained by polymerizing the monomer (a1), the monomer (a2) and optionally the monomer (a3), or a polymer obtained by introducing a polymerizable crosslinkable group into a polymer obtainable by polymerizing the monomer (a1), the monomer (a2') and optionally the monomer (a3).

The polymer (B) is preferably a polymer obtainable by polymerizing the monomer (a1) and optionally the monomer (a3).

The polymer (C) is preferably a polymer obtainable by polymerizing the monomer (a2) and optionally the monomer (a3), or a polymer obtained by introducing a polymerizable crosslinkable group into a polymer obtainable by polymerizing the monomer (a1), the monomer (a2') and optionally the monomer (a3).

The monomer (a1) is preferably a monomer made of an ester of a monool having a structure of $HO-R^1-R^F$ with an unsaturated monocarboxylic acid such as methacrylic acid, acrylic acid or $\alpha$-haloacrylic acid. As such a monomer, particularly preferred is a (meth)acrylate having a fluoroalkyl group of $-R^1-R^F$.

As specific monomers (a1), $CH_2=C(CH_3)COO-C_2H_4-(CF_2)_3CF_3$, $CH_2=CHCOO-C_2H_4-(CF_2)_3CF_3$, $CH_2=C(CH_3)COO-C_2H_4-(CF_2)_4CF_3$, $CH_2=CHCOO-C_2H_4-(CF_2)_4CF_3$, $CH_2=C(CH_3)COO-C_2H_4-(CF_2)_5CF_3$, $CH_2=CH-COO-C_2H_4-(CF_2)_5CF_3$, $CH_2=C(CH_3)COO-CH_2CF(CF_3)OCF_2CF_2CF_3$, $CH_2=CHCOO-CH_2CF(CF_3)OCF_2CF_2CF_3$, $CH_2=C(CH_3)COO-CH_2CF_2OCF_2CF_2OCF_3$, $CH_2=CHCOO-CH_2CF_2OCF_2CF_2OCF_3$, $CH_2=C(CH_3)COO-CH_2CF_2OCF_2CF_2OCF_2CF_3$, $CH_2=CHCOO-CH_2CF_2OCF_2CF_2OCF_2CF_3$, $CH_2=C(CH_3)COO-CH_2CF_2OCF_2CF_2OCF_2CF_2OCF_3$, $CH_2=CHCOO-CH_2CF_2OCF_2CF_2OCF_2CF_2OCF_3$, etc. may be mentioned. Among them, from the viewpoint that good liquid repellency is obtainable, $CH_2=C(CH_3)COO-C_2H_4-(CF_2)_5CF_3$ and $CH_2=C(CH_3)COO-CH_2CF_2OCF_2CF_2OCF_3$ are preferred.

As the monomer (a1), one type may be used alone, or two or more types may be used in combination.

As the polymerizable crosslinkable group in the monomer (a2), an epoxy group and oxetane group are preferred. As the monomer (a2), a (meth)acrylate having a polymerizable crosslinkable group may be mentioned.

As specific monomers (a2), glycidyl (meth)acrylate, 4-glycidyloxybutyl (meth)acrylate, (3-ethyloxetan-3-yl)methyl (meth)acrylate, 3,4-epoxycyclohexylmethyl (meth)acrylate, etc. may be mentioned. As the monomer (a2), one type may be used alone, or two or more types may be used in combination.

As the specific reactive site in the monomer (a2'), a hydroxy group and a carboxy group are preferred, and a hydroxy group is particularly preferred.

As the monomer (a2'), a (meth)acrylate having a hydroxy group is preferred.

Specific examples of the monomer having a hydroxy group as a specific reactive site of the monomer (a2') may be 2-hydroxyethyl (meth)acrylate, 2-hydroxypropyl (meth)acrylate, 3-hydroxypropyl (meth)acrylate, 4-hydroxybutyl (meth)acrylate, 5-hydroxypentyl (meth)acrylate, 6-hydroxyhexyl (meth)acrylate, 4-hydroxycyclohexyl (meth)acrylate, etc.

Specific examples of the monomer having a carboxy group as a specific reactive site of the monomer (a2') may be acrylic acid, methacrylic acid, vinyl acetate, etc.

As the monomer (a2'), one type may be used alone, or two or more types may be used in combination.

The compound (b) has a polymerizable crosslinkable group and a reactive functional group capable of bonding to a specific reactive site. As the polymerizable crosslinkable group in the compound (b), a group having an ethylenic double bond is preferred, and a (meth)acryloyloxy group and a vinyloxy group are more preferred. Particularly, an acryloyloxy group is preferred, since it is excellent in photocuring properties.

In a case where the specific reactive site in the monomer (a2') is a hydroxy group, the reactive functional group capable of bonding to the specific reactive site in the compound (b) may be an isocyanate group, an acyl chloride group, etc.

As the compound (b) having an isocyanate group and a polymerizable crosslinkable group, 2-(meth)acryloyloxyethyl isocyanate may be mentioned. As the compound (b) having an acyl chloride group and a polymerizable crosslinkable group, a (meth)acryloyl chloride may be mentioned.

In a case where the specific reactive site in the monomer (a2') is a carboxy group, the reactive functional group capable of bonding to the specific reactive site in the compound (b) may be an epoxy group. As the compound (b) having an epoxy group and a polymerizable crosslinkable group, glycidyl (meth)acrylate, 4-glycidyloxybutyl (meth)acrylate, 3,4-epoxycyclohexylmethyl (meth)acrylate, etc. may be mentioned.

The monomer (a3) is a monomer other than the monomers (a1), (a2) and (a2'). The monomer (a3) may, specifically, be methyl (meth)acrylate, ethyl (meth)acrylate, propyl (meth)acrylate, n-butyl (meth)acrylate, isobutyl (meth)acrylate, tert-butyl (meth)acrylate, hexyl (meth)acrylate, cyclohexyl (meth)acrylate, 2-ethylhexyl (meth)acrylate, octyl (meth)acrylate, decyl (meth)acrylate, isodecyl (meth)acrylate, lauryl (meth)acrylate, stearyl (meth)acrylate, benzyl (meth)acrylate, isobornyl (meth)acrylate, adamantyl (meth)acrylate, dimethylaminoethyl (meth)acrylate, methoxyethyl (meth)acrylate, methoxybutyl (meth)acrylate, butoxyethyl (meth)acrylate, phenoxyethyl (meth)acrylate, methoxy polyethylene glycol (meth)acrylate, ethoxy polyethylene glycol (meth)acrylate, propoxy polyethylene glycol (meth)acrylate, butoxy polyethylene glycol (meth)acrylate (provided that the number of oxyethylene groups in the polyethylene glycol chain is in a range of from 2 to 300), 2-{(meth)acryloyloxy}ethyl-2'-(trimethylammonio)ethyl phosphate, 3-{(meth)acryloyloxy}propyl-2'-(trimethylammonio)ethyl phosphate, 4-{(meth)acryloyloxy}butyl-2'-(trimethylammonio)ethyl phosphate, 5-{(meth)acryloyloxy}pentyl-2'-(trimethylammonio)ethyl phosphate, 6-{(meth)acryloyloxy}hexyl-2'-(trimethylammonio)ethyl phosphate, 2-{(meth)acryloyloxy}ethyl-2'-(triethylammonio)ethyl phosphate, 2-{(meth)acryloyloxy}ethyl-2'-(tripropylammonio)ethyl phosphate, 2-{(meth)acryloyloxy}ethyl-2'-(tributylammonio)ethyl phosphate, 2-{(meth)acryloyloxy}ethyl-2'-(tricyclohexylammonio)ethyl phosphate, 2-{(meth)acryloyloxy}ethyl-2'-(triphenylammonio)ethyl phosphate, 2-{(meth)acryloyloxy}ethyl-2'-(trimethanolammonio)ethyl phosphate, 2-{(meth)acryloyloxy}propyl-2'-(trimethylammonio)ethyl phosphate, 2-{(meth)acryloyloxy}butyl-2'-(trimethylammonio)ethyl phosphate, 2-{(meth)acryloyloxy}pentyl-2'-(trimethylammonio)ethyl phosphate, 2-{(meth)acryloyloxy}hexyl-2'-(trimethylammonio)ethyl phosphate, 2-(vinyloxy)ethyl-2'-(trimethylammonio)ethyl phosphate, 2-(allyloxy)ethyl-2'-(trimethylammonio)ethyl phosphate, 2-(p-vinylbenzyl)ethyl-2'-(trimethylammonio)ethyl phosphate, 2-(p-vinylbenzoyloxy)ethyl-2'-(trimethylammonio)ethyl phosphate, 2-(styryloxy)ethyl-2'-(trimethylammonio)ethyl phosphate, 2-(p-vinylbenzyl)ethyl-2'-(trimethylammonio)ethyl phosphate, 2-(vinyloxycarbonyl)ethyl-2'-(trimethylammonio)ethyl phosphate, 2-(allyloxycarbonyl)ethyl-2'-(trimethylammonio)ethyl phosphate, 2-(acryloylamino)ethyl-2'-(trimethylammonio)ethyl phosphate, 2-(vinyl carbonylamino)ethyl-2'-(trimethylammonio)ethyl phosphate, ethyl-(2'-trimethylammonioethylphosphorylethyl) fumarate, butyl-(2'-trimethylammonioethylphosphorylethyl)fumarate, hydroxyethyl-(2'-trimethylammonioethylphosphorylethyl)fumarate, ethyl-(2'-trimethylammonioethylphosphorylethyl)malate, butyl-(2'-trimethylammonioethylphosphorylethyl)malate, or hydroxyethyl-(2'-trimethylammonioethylphosphorylethyl)malate.

As the monomer (a3), one type may be used alone, or two or more types may be used in combination.

The proportion of the structural unit (u1) to all structural units in the polymer (A), is preferably from 10 to 90 mol%, more preferably from 20 to 90 mol%. The proportion of the structural unit (u2) is preferably from 10 to 90 mol%, more preferably from 10 to 80 mol%. The proportion of the structural unit (u3) is preferably from 0 to 80 mol%, more preferably from 0 to 60 mol%. In particular, the ratio of the respective structural units to all structural units in the polymer (A) is preferably such that the structural unit (u1) is from 20 to 90 mol%, the structural unit (u2) is from 10 to 80 mol%, and the structural unit (u3) is from 0 to 60 mol%.

The proportion of the structural unit (u1) to all structural units in the polymer (B) is preferably from 10 to 100 mol%, more preferably from 20 to 100 mol%. The proportion of the structural unit (u3) is preferably from 0 to 90 mol%, more preferably from 0 to 80 mol%.

The proportion of the structural unit (u2) to all structural units in the polymer (C) is preferably from 10 to 100 mol%, more preferably from 20 to 100 mol%. The proportion of the structural unit (u3) is preferably from 0 to 90 mol%, more preferably from 0 to 80 mol%.

The proportions of the respective structural units in the polymer can be obtained, for example, from the integration ratio of the peaks specific to the respective units in the $^1$H-NMR.

The weight average molecular weight of each of the polymers (A), (B) and (C) is not particularly limited, but is preferably from 5,000 to 500,000, more preferably from 10,000 to 200,000. When the weight average molecular weight is within this range, processing by lithography can be stably done.

The weight average molecular weight of the polymer is a value calculated as the standard polymethyl methacrylate as measured by the gel permeation chromatography (GPC).

As a method for producing each of the polymers (A), (B) and (C), solution polymerization, bulk polymerization, emulsion polymerization, etc. may be mentioned. Among them, solution polymerization is preferred.

In order to adjust the weight average molecular weight, a chain transfer agent may be used at the time of the polymerization.

As the chain transfer agent, dodecanethiol, octadecanethiol, thioglycerol, 2-ethyl hexanethiol, cyclohexyl mercaptan, furfuryl mercaptan, benzylthiol, cyclohexyl methanethiol, 2,4,4-trimethyl-1-pentanethiol, tert-nonyl mercaptan, etc. may be mentioned. From the viewpoint of being readily available, dodecanethiol, octadecanethiol, thioglycerol, 2-ethyl hexanethiol, cyclohexyl mercaptan, furfuryl mercaptan and benzylthiol are preferred, and from the viewpoint of low odor, thioglycerol is particularly preferred.

The photoinitiator according to the present invention has an absorption coefficient at wavelength of 365 nm of at most 400 [mL·g$^{-1}$·cm$^{-1}$]. When such an initiator is used, processing by lithography will be possible, and light as noise during fluorescence analysis will be suppressed. The light to be noise is specifically light that is observed as fluorescence when measured by a laser-excited fluorescence spectrometer. The cause of such light is not clearly understood, but it is considered to be autofluorescence or stray light due to scattering.

The absorption coefficient at a wavelength of 365 nm of the photoinitiator is at most 400 [mL·g$^{-1}$·cm$^{-1}$], but more preferably at most 200 [mL·g$^{-1}$·cm$^{-1}$]. Further, the absorption coefficient is preferably at least 1 [mL·g$^{-1}$·cm$^{-1}$], more preferably at least 10 [mL·g$^{-1}$·cm$^{-1}$].

As the photoinitiator, a photoradical generator or a photo-acid generator may be mentioned.

Specific examples of such a photo-radical generator may be 2,2-dimethoxy-1,2-diphenylethan-1-one, 2-hydroxy-1-phenylacetophenone, methyl phenyl ketone, dibenzoyl, benzophenone, benzoin isopropyl ether, 2-hydroxy-2-methyl-1-phenyl-propan-1-one, 1-[4-(2-hydroxyethoxy)-phenyl]-2-hydroxy-2-methyl-1-propan-1-one, 1-hydroxycyclohexyl-phenyl-ketone, 2-hydroxy-1-{4-[4-(2-hydroxy-2-methyl-propionyl)-benzyl]phenyl}-2-methyl-propan-1-one, etc. Among them, 2,2-dimethoxy-1,2-diphenylethan-1-one, 2-hydroxy-2-methyl-1-phenyl-propan-1-one, 1-hydroxycyclohexyl-phenyl-ketone, or 2-hydroxy-1-{4-[4-(2-hydroxy-2-methyl-propionyl)-benzyl]phenyl}-2-methyl-propan-1-one, is preferred, since the fluorescence of the obtainable coating film will be small, and it will be excellent in photocurable performance. One of these photo-radical generators may be used alone, or two or more of them may be used as mixed.

Specific tradenames of photoradical generators may, for example, be IRGACURE (trademark) 651 (manufactured by BASF), IRGACURE 184 (manufactured by BASF), IRGACURE 127 (manufactured by BASF), IRGACURE 500 (manufactured by BASF), IRGACURE 2959 (manufactured by BASF), 1173 (manufactured by BASF), etc.

As the photo-acid generator, a triarylsulfonium salt, a diaryliodonium salt, a sulfonyl diazomethane, etc. may be mentioned.

Specific examples of the cation moiety of the triarylsulfonium salt or the diaryliodonium salt may be triphenylsulfonium, diphenyl-4-methylphenylsulfonium, tris(4-methylphenyl)sulfonium, diphenyl-2,4,6-trimethylphenylsulfonium, 4-(phenylthio)phenyldiphenylsulfonium, diphenyliodonium, 4-isopropyl-4'-methyldiphenyliodonium, 4-methyl-4'-methylpropyldiphenyliodonium, bis(4-tert-butylphenyl)iodonium, 4-methoxyphenyphenyliodonium, etc. Examples of the anionic moiety may, for example, be trifluoromethanesulfonate, nonafluorobutanesulfonate, hexafluorophosphate, tetrafluoroborate, tris(pentafluoroethyl)trifluorophosphate, tris(heptafluoropropyl)trifluorophosphate, tris(nonafluoroisobutyl)trifluorophosphate, bis(nonafluoroisobutyl)tetrafluorophosphate, etc.

Specific examples of the sulfonyl diazomethane may be bis(phenylsulfonyl) diazomethane, bis(tert-butylsulfonyl) diazomethane, bis(cyclohexylsulfonyl) diazomethane, bis(p-toluenesulfonyl) diazomethane, etc.

Among them, 4-(phenylthio)phenyldiphenylsulfonium tris(heptafluoropropyl)trifluorophosphate, 4-(phenylthio)phenyldiphenylsulfonium tris(nonafluoroisobutyl)trifluorophosphate, 4-(phenylthio)phenyldiphenylsulfonium bis(nonafluoroisobutyl)tetrafluorophosphate, 4-(phenylthio)phenyldiphenylsulfonium (pentafluoroethyl)trifluorophosphate, diphenyl-2,4,6-trimethylphenylsulfonium trifluoromethanesulfonate, diphenyl-2,4,6-trimethylphenylsulfonium nonafluorobutanesulfonate, bis(phenylsulfonyl)diazomethane, or bis(p-toluenesulfonyl)diazomethane, is preferred, since fluorescence of the obtainable coating film will be small, and it will be excellent in photocuring properties.

One of these photo-acid generators may be used alone, or two or more of them may be used as mixed.

Specific trade names of photo-acid generators may be IRGACURE 250 (manufactured by BASF), CPI (trademark)-100P (manufactured by San-Apro Ltd.), CPI-210S (manufactured by San-Apro Ltd.), WPAG199 (manufactured by Wako Pure Chemical Industries, Ltd.), etc.

The photosensitive composition of the present invention comprises the above polymers and the above photoinitiator, and may optionally contain a component other than them, such as a solvent, etc.

The photosensitive composition of the present invention preferably contains a solvent. Such a solvent may be one which dissolves or disperses the polymers and the photoinitiator, and one capable of dissolving them is more preferred.

Here, as described above, the volatile component such as the solvent is a component to be removed before irradiation with light.

The solvent may be a fluorinated solvent or a non-fluorinated solvent.

As specific fluorinated solvents, as ASAHIKLIN (trademark) manufactured by Asahi Glass Company, Limited, 1H-tridecafluorohexane (AC2000), 1,1,1,2,2,3,3,4,4,5,5,6,6-tridecafluorooctane (AC6000), 1,1,2,2-tetrafluoro-1-(2,2,2-trifluoroethoxy)ethane (AE3000), dichloropentafluoropropane (AK-225), etc. may be exemplified. Further, as others, Cytop (trademark) CT-solv100E (manufactured by Asahi Glass Company, Limited), 1-methoxynonafluorobutane (manufactured by 3M Japan Limited, Novec (trademark) 7100), 1-ethoxy-nonafluorobutane (manufactured by 3M Japan Limited, Novec (trademark) 7200), 1,1,1,2,3,3-hexafluoro-4-(1,1,2,3,3,3-hexafluoropropoxy)pentane (manufactured by 3M Japan Limited, Novec (trademark) 7600), 2H,3H-perfluoropentane (manufactured by Du Pont-Mitsui Fluorochemicals Company, Ltd., Vertrel (trademark) XF), 3,3,4,4,5,5,6,6,7,7,8,8,8-tridecafluoro-1-octanol, 4,4,5,5,6,6,7,7,8,8,9,9,9-tridecafluoro-1-nonanol, hexafluorobenzene, hexafluoro-2-propanol, 2,2,3,3,4,4,5,5-octafluoro-1-pentanol, 1H,1H,7H-dodecafluoro-1-heptanol, etc. may be mentioned.

As specific non-fluorinated solvents, a ketone such as cyclopentanone, cyclohexanone, methyl amyl ketone, 2-butanone, etc., an ester such as ethyl lactate, methyl benzoate, ethyl benzoate, benzyl benzoate, methyl cellosolve acetate, ethyl cellosolve acetate, propylene glycol monomethyl ether acetate, propylene glycol monoethyl ether acetate, propylene carbonate, etc., an ether such as tetrahydrofuran, dioxane, dimethoxyethane, diethoxyethane, anisole, diglyme, triglyme, etc. may be mentioned.

Among them, propylene glycol monomethyl ether acetate, propylene glycol monoethyl ether acetate, hexafluoro-2-propanol, 2,2,3,3,4,4,5,5-octafluoro-1-pentanol, or 1H,1H,7H-dodecafluoro-1-heptanol is preferred, since the solubility is high, the boiling point is high, and the film-forming property is excellent.

One of these solvents may be used alone, or two or more of them may be used as mixed.

As a component other than a solvent, which the photosensitive composition of the present invention may contain, a radical crosslinking agent, an acid crosslinking agent, an adhesion imparting agent, or other additives, may be mentioned.

The radical crosslinking agent may be a compound having at least two ethylenic double bonds. Particularly preferred is a compound having from 2 to 6 acryloyloxy groups.

As specific radical crosslinking agents, diethylene glycol di(meth)acrylate, tetraethylene glycol di(meth)acrylate, tripropylene glycol di(meth)acrylate, neopentyl glycol di(meth)acrylate, trimethylolpropane tri(meth)acrylate, pentaerythritol tetra(meth)acrylate, 1,10-decanediol di(meth)acrylate, 2,2,3,3,4,4,5,5,6,6,7,7,8,8,9,9 hexadecafluoro-1,10-decanediol di(meth)acrylate, 2,2,3,3,4,4,5,5,6,6,7,7-dodecafluoro-1,8-octanediol di(meth)acrylate, 2,2,3,3,4,4,5,5-decafluoro-1,6-hexanediol di(meth)acrylate, $(CH_2=CHCOO-CH_2)_2C(CH_3)NHCOO-CH_2-(CF_2)_4-CH_2-OCON-HC(CH_3)(CH_2OCOCH=CH_2)_2$, $(CH_2=CHCOO-CH_2)_2C(CH_3)NHCOO-CH_2-(CF_2)_6-CH_2-OCON-HC(CH_3)(CH_2OCOCH=CH_2)_2$, $(CH_2=CHCOO-CH_2)_2C(CH_3)NHCOO-CH_2-(CF_2)_8-CH_2-OCON-HC(CH_3)(CH_2OCOCH=CH_2)_2$, etc. may be mentioned.

Among them, trimethylolpropane tri(meth)acrylate, pentaerythritol tetra(meth)acrylate, $(CH_2=CHCOO-CH_2)_2C(CH_3)NHCOO-CH_2-(CF_2)_4-CH_2-OCONHC(CH_3)(CH_2OCOCH=CH_2)_2$, $(CH_2=CHCOO-CH_2)_2C(CH_3)NHCOO-CH_2-(CF_2)_6-CH_2-OCONHC(CH_3)(CH_2OCOCH=CH_2)_2$, or $(CH_2=CHCOO-CH_2)_2C(CH_3)NHCOO-CH_2-(CF_2)_3-CH_2-OCONHC(CH_3)(CH_2OCOCH=CH_2)_2$, is preferred, since the compatibility with a resin is high, and the curing property is also high.

The acid crosslinking agent may be a compound having a group crosslinkable by the action of an acid. Specific acid crosslinking agents may, for example, be hexamethylolmethoxy methylmelamine, 1,3,4,6-tetrakis(methoxymethyl) glycoluril, 4,5-dimethoxy-1,3-bis(methoxymethyl)imidazolidin-1-one, 1,3-bis(methoxymethyl) urea, 3,4-epoxycyclohexylmethyl-3,4-epoxycyclohexane carboxylate, bis(2,3-epoxycyclopentyl) ether, ethylene glycol diglycidyl ether, 1,4-butanediol diglycidyl ether, 3-ethyl-3-hydroxymethyl oxetane, 3-ethyl[(3-ethyloxetan-3-yl)methoxy]methyl oxetane, diethylene glycol monovinyl ether, triethylene glycol divinyl ether, cyclohexyl divinyl ether, 3-(3,3,4,4,5,5,6,6,7,7,8,8,8-tridecafluorooctyloxy)-1,2-epoxypropane, 2,2,3,3,4,4,5,5,5-nonafluoropentyloxysilane, etc.

As the adhesion imparting agent, a coupling agent such as a silane coupling agent may be used. As the silane coupling agent, tetraethoxysilane, 3-glycidoxypropyltrimethoxysilane, 3-methacryloyloxypropyltrimethoxysilane, heptadecafluorooctyltrimethoxysilane, 3-chloropropyltrimethoxysilane, vinyltrichlorosilane, vinyltrimethoxysilane, etc. may be mentioned.

The content of the above-mentioned polymer(s) in the photosensitive composition is preferably from 5 to 70 mass%, more preferably from 10 to 60 mass%, further preferably from 10 to 50 mass%, particularly preferably from 10 to 40 mass%.

In a case where the polymers are composed of a combination of the polymer (B) and the polymer (C), the proportion of the polymer (B) to the total of the polymer (B) and the polymer (C) is preferably from 10 to 95 mass%, more preferably from 25 to 90 mass%.

The content of the photoinitiator in the photosensitive composition is preferably from 0.1 to 10 mass%, more preferably from 0.5 to 8 mass%, particularly preferably from 0.5 to 5 mass%.

The content of other components in the photosensitive composition is preferably from 0 to 20 mass%, more preferably from 0 to 10 mass%.

The proportion of the solvent in the photosensitive composition is the rest excluding the polymers, the photoinitiator and other components from the photosensitive composition.

The photosensitive composition is preferably a composition comprising, based on the entire composition, from 10 to 60 mass% of the polymer(s), from 1 to 10 mass% of the photoinitiator, from 30 to 89 mass% of a solvent and from 0 to 59 mass% of other components other than the solvent, more preferably a composition comprising from 10 to 40 mass% of the polymer(s), from 1 to 4 mass% of the photoinitiator, from 46 to 89 mass% of a solvent and from 0 to 10 mass% of other components.

The method for producing a biochip for fluorescence analysis of the present invention, is a method which comprises applying the photosensitive composition on a liquid contact surface of a substrate, followed by exposure and development to form a liquid-repellent film.

By applying the photosensitive composition on the liquid contact surface of the substrate, and when a volatile component such as a solvent is contained, by removing the volatile component, a photosensitive film will be formed. When this photosensitive film is subjected to selective exposure, at the exposed portions of the photosensitive film, a radical or an acid will be generated from the photoinitiator, and by its action, polymerizable crosslinkable groups will be crosslinked (cured), whereby the solubility of the exposed portions to the developer will decrease. Therefore, when the photosensitive film after the exposure is developed by a developer, unexposed portions will be dissolved in the developer and removed, whereas the exposed portions will remain without being removed. Thus, a liquid-repellent film (cured film) having through-holes formed in the thickness direction, will be formed, to obtain a biochip for fluorescence analysis, comprising the substrate and the liquid-repellent film formed on a surface of the substrate. Holes to be formed in the liquid-repellent film are typically in a plurality.

Coating

As the substrate to be coated with the photosensitive composition of the present invention, the material is not particularly limited so long as its fluorescence intensity is small. For example, various glass plates, or thermoplastic plastic sheets of e.g. polypropylene, polyethylene, polycarbonate, polymethyl methacrylate, polystyrene, etc., may be mentioned. From the viewpoint of heat resistance, a glass plate, particularly a quartz glass plate is preferably used.

As the substrate, a substrate having a lyophilic surface is preferred. As the substrate having a lyophilic surface, a substrate made of a lyophilic material, such as the above-mentioned various glass plates, thermoplastic plastic sheets, etc., may be mentioned. Otherwise, it may be a substrate having a lyophilic film. As the lyophilic surface, preferred is a surface having a contact angle of water of less than 50 degrees, or a contact angle of propylene glycol monomethyl ether acetate of less than 20 degrees. The above-mentioned liquid-repellent film is formed on this lyophilic surface, and the bottom surface of the holes in the liquid-repellent film is preferably constituted by the lyophilic surface.

As the coating method, a spraying method, a roll coating method, a spin coating method or a bar coating method may, for example, be mentioned.

The wet coating film obtained by applying the composition containing a solvent is preferably baked (hereinafter referred to as prebaking). By the pre-baking, the solvent is swiftly volatilized, whereby a dried coating film (photosensitive film) having no fluidity will be obtained. The prebaking conditions may vary depending upon e.g. the types, blend proportions, etc. of the respective components, but are preferably from 50 to 120°C and from about 10 to 2,000 seconds.

The film thickness of the dried coating film is not particularly limited, but is preferably from 0.1 to 20 $\mu$m, more preferably from 0.5 to 10.0 $\mu$m.

Exposure

The dried coating film is subjected to exposure through a mask having a predetermined pattern. Light to be used for the exposure is a light with a wavelength of from 100 to 500 nm, preferably a light of from 200 to 450 nm, particularly preferably an i-line (365 nm). The irradiation apparatus is preferably one wherein a high-pressure mercury lamp, (UV) LED, a laser or the like is used as a light source. The exposure dose is preferably within a range of from 5 to 2,000 mJ/cm$^2$.

After the exposure, as the case requires, the coating film after the exposure is preferably subjected to post-exposure baking (PEB) treatment to promote the crosslinking reaction. The PEB treatment conditions are preferably within ranges of from 50 to 150°C and from 10 to 2,000 seconds.

Development

The exposed dried coating film is developed by a developer to remove non-exposed portions. The developer is not particularly limited, but it is preferred to use a solvent such as one mentioned above as the solvent. The time for the development is preferably from 5 to 180 seconds. Further, the developing method may be any one of a puddle method, a dipping method, a shower method, etc.

After the development, the substrate is dried to obtain a patterned coating film (liquid-repellent film) having exposed portions of the dried coating film cured and having unexposed portions removed. The obtained patterned coating film is preferably cured at from 120 to 250°C for from 5 to 90 minutes by a heating device such as a hot plate or oven to promote crosslinking.

The finally obtainable liquid-repellent film (cured film) has a low fluorescence and is excellent in liquid repellency.

The fluorine content in the liquid-repellent film (the content of fluorine atoms in the film) is preferably from 15 to 75 mass%, more preferably from 15 to 60 mass%. When the fluorine content is at least the lower limit value in the above range, the liquid repellency is more excellent. When the fluorine content is at most the upper limit value in the above range, the solubility of unexposed portions in the developer is more excellent at the time of development. The fluorine content in the liquid-repellent film may be adjusted by the fluorine contents in the polymers, the proportions of the polymers in the photosensitive composition, etc.

The fluorine content of the liquid-repellent film is calculated from the fluorine contents in the polymers, the proportions of the polymers in the liquid-repellent film, etc. The fluorine content in a polymer is measured by the method described in Examples given later. The fluorine content in the polymer may be adjusted by the content of a fluoroalkyl group (the proportion of the structural unit having a fluoroalkyl group).

By the above method, a biochip for fluorescence analysis is produced.

In the biochip for fluorescence analysis, the liquid-repellent film has a fluorescence intensity of preferably at most 15,000, more preferably at most 10,000, further preferably at most 5,000, particularly preferably at most 4,000, as measured by the measuring method described in Examples given later. When its fluorescence intensity is at most the above upper limit value, it is useful for fluorescence analysis.

The fluorescence intensity may be adjusted by the content of the photoinitiator.

The liquid-repellent film preferably has a contact angle of water of at least 90 degrees or a contact angle of propylene glycol monomethyl ether acetate of at least 45 degrees, particularly preferably has a contact angle of water of at least 95 degrees or a contact angle of propylene glycol monomethyl ether acetate of at least 47 degrees. When such a contact angle is at least the above lower limit value, the liquid repellency will be excellent enough.

The contact angle is measured by the method described in Examples given later.

The contact angle may be adjusted by the fluorine content in the liquid-repellent film.

The liquid-repellent film has a protein adsorption rate Q of preferably at most 5%, particularly preferably at most 1%, as measured by the measuring method described in Examples given later. When the protein adsorption rate Q is at most 5%, the low cell adhesion will be excellent.

According to the present invention, it is possible to obtain a biochip for fluorescence analysis having a liquid-repellent film, of which the fluorescence intensity is at most 15,000, the contact angle of the water is at least 90 degrees, and the protein adsorption rate Q is at most 5%.

The fluorescence analysis using the above biochip for fluorescence analysis, is preferably carried out by using a fluorescence microscope and a microarray scanner. By irradiating a light beam to the biochip for fluorescence analysis, the fluorescence thereby emitted, is measured. It is preferred to use a laser beam as the excitation light source. The excitation wavelength is, in order to excite a fluorescent dye to be commonly used, preferably between 450 nm to 800 nm, and a wavelength of 488 nm, 532 nm, 594 nm, 635 nm or 650 nm is preferred. Particularly preferred is a wavelength of 532 nm or 635 nm. For the measurement of the emitted fluorescence, it is preferred to use a photomultiplier tube.

EXAMPLES

The present invention will be described in detail with reference to the following Examples, but the present invention is not limited thereto.

[Raw materials, etc.]

Compounds represented by the following abbreviations were used as raw materials, etc.

(Monomers)

C6FMA: $CH_2=C(CH_3)COO-C_2H_4-(CF_2)_4CF_3$ (produced by the method described in Example 1 of JP-A-2004-359616).

FPEG2MA: $CH_2=C(CH_3)COO-CH_2CF_2OCF_2CF_2OCF_3$.

GMA: glycidyl methacrylate.

ECHMA: 3,4-epoxycyclohexylmethyl methacrylate (manufactured by Daicel Corporation, Cyclomer M100).

OXMA: (3-ethyloxetan-3-yl)methyl methacrylate (manufactured by Osaka Organic Chemical Industry Ltd., OXE-30).

HEMA: 2-hydroxyethyl methacrylate.

AOI: 2-isocyanatoethyl acrylate (manufactured by Showa Denko K.K., Karenz AOI).

PEG9MA: methoxy polyethylene glycol methacrylate (manufactured by NOF Corporation, Blemmer PME400), number of oxyethylene groups: 9.

MPCMA: 2-{methacryloyloxy}ethyl-2'-(trimethylammonio)ethyl phosphate (manufactured by Aldrich).

(Solvents)

MEK: 2-butanone.

OFPO: 2,2,3,3,4,4,5,5-octafluoro-1-pentanol.

PGMEA: propylene glycol monomethyl ether acetate.

(Polymerization initiator)

V65: 2,2'-azobis(2,4-dimethylvaleronitrile) (manufactured by Wako Pure Chemical Industries, Ltd., V65).

(Photoinitiators)

WPAG199: bis(p-toluenesulfonyl)diazomethane (manufactured by Wako Pure Chemical Industries, Ltd., WPAG199).

CP1210S: photo-acid generator (manufactured by San-Apro Ltd., CPI-210S).

CPI310B: photo-acid generator (manufactured by San-Apro Ltd., CPI-310B).

CPI410S: photo-acid generator (manufactured by San-Apro Ltd., CPI-410S).

IRPAG103: 2-[2-(propylsulfonyloxyimino)thiophen-3(2H)-ylidene]-2-(2-methylphenyl)acetonitrile (manufactured by BASF, IRGACURE (trademark) PAG103).

OXE01: 1,2-octanedione-1-[4-(phenylthio)-2-(o-benzoyl oxime)] (manufactured by BASF, IRGACURE (trademark) OXE01).

IR907: 2-methyl-1-(4-methylthiophenyl)-2-morpholinopropan-1-one (manufactured by BASF, IRGACURE (trademark) 907).

IR184: 1-hydroxycyclohexyl-phenyl-ketone (manufactured by BASF, IRGACURE (trademark) 184).

IR127: 2-hydroxy-1-{4-[4-(2-hydroxy-2-methyl-propionyl)-benzyl]phenyl}-2-methyl-propan-1-one (manufactured by BASF, IRGACURE (trademark) 127).

IR819: bis(2,4,6-trimethylbenzoyl)-phenylphosphine oxide (manufactured by BASF, IRGACURE (trademark) 819).

(Chain transfer agents)

Thioglycerol (manufactured by Tokyo Chemical Industry Co., Ltd.)

Dodecanethiol (manufactured by Kanto Chemical Co., Inc.).

[Physical properties and evaluations]

($^1$H-NMR)

$^1$H-NMR spectra of a compound were measured by using FT-NMR apparatus (manufactured by JEOL Ltd., JNM-AL300).

(Weight average molecular weight)

The weight average molecular weight (Mw) and number average molecular weight (Mn) of the polymer in each of Synthesis Examples 1 to 18 were obtained by using a calibration curve prepared by using a standard polymethyl methacrylate sample with a known molecular weight, from the chromatogram obtained by a high-speed gel permeation chromatography (GPC) apparatus (manufactured by TOSOH CORPORATION, HLC-8220).

(Fluorine content)

The "fluorine content" is the proportion (mass%) of fluorine atoms in a polymer (100 mass%), and is obtainable by the following formula.

$$Q_F = [19 \times N_F / M_A] \times 100$$

wherein $Q_F$ is a fluorine content, $N_F$ is the sum of values obtained, for every type of units constituting the polymer, by multiplying the number of fluorine atoms in the unit, by the molar ratio of the unit to all units, and $M_A$ is the sum of values obtained, for every type of units constituting the polymer, by multiplying the total of the atomic weights of all atoms constituting the unit, by the molar ratio of the unit to all units.

(Synthesis Example 1)

56.6 g of C6FMA, 43.4 g of GMA and 1.62 g of V65 were dissolved in 400 g of MEK, followed by stirring under a nitrogen atmosphere at 50°C for 24 hours. The reaction liquid was added to 5,000 mL of hexane, and the precipitated solid was filtered through a polytetrafluoroethylene (PTFE) filter having a pore size of 3 μm, to obtain a polymer. The molecular weight (Mw), molecular weight distribution (Mw/Mn) and copolymerization ratio of the obtained polymer are shown in Table 3. Here, the copolymerization ratio was calculated from the signals at 4.3 ppm and 3.8 ppm of $^1$H-NMR (d-Acetone, TMS).

(Synthesis Examples 2 to 12)

The polymerization was carried out in the same manner as in Synthesis Example 1. The amounts (g) of raw materials, etc. used in the reaction are shown in Table 1. Further, the molecular weight (Mw), molecular weight distribution (Mw/Mn) and copolymerization ratio of the obtained polymer are shown in Table 3. The copolymerization ratio was calculated, in each of Synthesis Examples 2 to 4 and 10 to 12, from the signals at 4.3 ppm and 3.8 ppm of $^1$H-NMR (d-Acetone, TMS), in each of Synthesis Examples 5 to 7, from the signals at 4.6 ppm and 4.0 ppm of $^1$H-NMR (hexafluorobenzene, p-hexafluoroxylene 8.0 ppm), in Synthesis Example 8, from the signals at 4.6 ppm and 4.0 ppm of H-NMR (hexafluorobenzene, p-hexafluoroxylene 8.0 ppm) and in Synthesis Example 9, from the signals at from 4.3 to 4.6 ppm and 3.0 ppm of H-NMR (hexafluorobenzene, p-hexafluoroxylene 8.0 ppm).

(Synthesis Example 13)

2.0 g of the polymer obtained in Synthesis Example 11, 0.90 g of AOI, 3.6 mg of dibutyltin dilaurate and 45 mg of benzohydroxytoluene were dissolved in 18 g of MEK and shaken at 40°C for 24 hours. The reaction liquid was added to 600 mL of hexane, and the precipitated solid was filtered through a PTFE filter having a pore size of 3 $\mu$m, to obtain a polymer.

(Synthesis Example 14)

2.0 g of the polymer obtained in Synthesis Example 12, 0.50 g of AOI, 2.0 mg of dibutyltin dilaurate and 25 mg of benzohydroxytoluene were dissolved in 18 g of MEK and shaken at 40°C for 24 hours. The reaction liquid was added to 600 mL of hexane, and the precipitated solid was filtered through a PTFE filter having a pore size of 3 $\mu$m, to obtain a polymer.
The molecular weight (Mw), molecular weight distribution (Mw/Mn) and copolymerization ratio of the polymer synthesized in each of Synthesis Example 13 and 14 are shown in Table 3. Here, the copolymerization ratio was calculated from the signals at 4.3 ppm and 3.8 ppm of $^1$H-NMR (d-Acetone, TMS).

(Synthesis Example 15)

The polymerization was carried out in the same manner as in Synthesis Example 1. The amounts (g) of raw materials, etc. used in the reaction are shown in Table 2. Further, the molecular weight (Mw), molecular weight distribution (Mw/Mn) and copolymerization ratio of the obtained polymer are shown in Table 4. The copolymerization ratio was calculated from the signals at 4.3 ppm, 3.8 ppm and 3.6 ppm of $^1$H-NMR (d-Acetone, TMS).

(Synthesis Example 16)

The polymerization was carried out in the same manner as in Synthesis Example 1. The amounts (g) of raw materials, etc. used in the reaction are shown in Table 2. Further, the molecular weight (Mw), molecular weight distribution (Mw/Mn) and copolymerization ratio of the obtained polymer are shown in Table 4. The copolymerization ratio was calculated from the signals at 3.7 ppm, 2.9 ppm and 2.7 ppm of $^1$H-NMR (d-methanol, TMS).

(Synthesis Example 17)

2.0 g of C6FMA, 1.53 g of GMA, 0.034 g thioglycerol and 0.134 g of V65 were dissolved in 8.23g of PGMEA, and after nitrogen substitution, shaken at 50°C for 24 hours. Then, the temperature was raised to 70°C, followed by shaking for 2 hours. 0.70 g of the reaction liquid was added to 50 mL of hexane, and the precipitated solid was filtered through a PTFE filter having a pore size of 3 $\mu$m, to obtain a polymer. The molecular weight (Mw), molecular weight distribution (Mw/Mn) and copolymerization ratio of the obtained polymer are shown in Table 3. Here, the copolymerization ratio was calculated from the signals at 4.3 ppm and 3.8 ppm of $^1$H-NMR (d-Acetone, TMS), and the thioglycerol residue ($-SCH_2CH(OH)CH_2OH$) at the main chain terminal was calculated from 3.54 ppm.

(Synthesis Example 18)

The polymerization was carried out in the same manner as in Synthesis Example 17 except that as a chain transfer agent, 0.062 g of dodecanethiol was used in place of 0.034 g of thioglycerol. The amounts (g) of raw materials, etc. used in the reaction are shown in Table 1. Further, the molecular weight (Mw), molecular weight distribution (Mw/Mn) and copolymerization ratio of the obtained polymer are shown in Table 3. The copolymerization ratio was calculated from the signals at 3.7 ppm, 2.9 ppm and 2.7 ppm of $^1$H-NMR (d-methanol, TMS), and the dodecanethiol residue ($-S(CH_2)_{11}CH_3$) at the main chain terminal was calculated from 2.54 ppm.

(Synthesis Example 19)

9.0 g of C6FMA, 0.053 g of thioglycerol and 0.181 g of V65 were dissolved in 20.97 g of AC6000, and after nitrogen substitution, shaken at 50°C for 24 hours. Then, the temperature was raised to 70°C, followed by shaking for 2 hours. The reaction liquid was added to 500 mL of hexane, and the precipitated solid was filtered through a PTFE filter having a pore size of 3 $\mu$m, to obtain a polymer. The molecular weight (Mw), molecular weight distribution (Mw/Mn) and copolymerization ratio of the obtained polymer are shown in Table 3. Here, the copolymerization ratio was calculated from the signals at 4.3 ppm and 3.8 ppm of $^1$H-NMR (d-Acetone, TMS), and the thioglycerol residue ($-SCH_2CH(OH)CH_2OH$) at the main chain terminal was calculated from 3.54 ppm.

(Synthesis Example 20)

8.2 g of GMA, 0.13 g of thioglycerol and 0.502 g of V65 were dissolved in 19.06 g of MEK, and after nitrogen substitution, shaken at 50°C for 24 hours. Then, the temperature was raised to 70°C, followed by shaking for 2 hours. The reaction liquid was added to 500 mL of hexane, and the precipitated solid was filtered through a PTFE filter having a pore size of 3 $\mu$m, to obtain a polymer. The molecular weight (Mw), molecular weight distribution (Mw/Mn) and copolymerization ratio of the obtained polymer are shown in Table 3. Here, the copolymerization ratio was calculated from the signals at 4.3 ppm and 3.8 ppm of $^1$H-NMR (d-Acetone, TMS), and the thioglycerol residue ($-SCH_2CH(OH)CH_2OH$) at the main chain terminal was calculated from 3.54 ppm.

[Table 1]

| Synthesis Example | (a1) | | (a2) | | | (a2') | Initiator | Chain transfer agent | Solvent | | |
|---|---|---|---|---|---|---|---|---|---|---|---|
| | C6FMA | FPEG 2MA | GMA | ECH MA | OXMA | HEMA | V65 | | AK-225 | MEK | PGMEA |
| 1 | 56.6 | - | 43.4 | - | - | - | 1.62 | Absent | - | 400 | - |
| 2 | 1.70 | - | 1.30 | - | - | - | 0.11 | Absent | - | 27 | - |
| 3 | 5.09 | - | 3.91 | - | - | - | 0.098 | Absent | - | 21 | - |
| 4 | 5.09 | - | 3.91 | - | - | - | 0.049 | Absent | - | 21 | - |
| 5 | 3.01 | - | 0.99 | - | - | - | 0.052 | Absent | 36.0 | - | - |
| 6 | 3.50 | - | 0.50 | - | - | - | 0.043 | Absent | 36.0 | - | - |
| 7 | 3.86 | - | 0.14 | - | - | - | 0.037 | Absent | 36.0 | - | - |
| 8 | 2.75 | - | - | 1.25 | - | - | 0.05 | Absent | 38.5 | - | - |
| 9 | 2.80 | - | - | - | 1.20 | - | 0.05 | Absent | 36.0 | - | - |
| 10 | - | 2.06 | 1.95 | - | - | - | 0.073 | Absent | 36.0 | - | - |
| 11 | 2.06 | - | - | - | - | 1.44 | 0.059 | Absent | - | 31.5 | - |
| 12 | 2.69 | - | - | - | - | 0.81 | 0.046 | Absent | - | 31.5 | - |
| 17 | 2.00 | - | 1.53 | - | - | - | 0.134 | Present | - | - | 8.23 |
| 18 | 2.00 | - | 1.53 | - | - | - | 0.134 | Present | - | - | 8.18 |

[Table 2]

| Synthesis Example | (a1) | (a2) | (a3) | | Initiator | Solvent |
|---|---|---|---|---|---|---|
| | C6FMA | GMA | MPCMA | PEG9MA | V65 | MEK |
| 15 | 1.0 | 0.22 | - | 1.9 | 0.019 | 28.2 |
| 16 | 1.2 | 0.26 | 1.4 | - | 0.023 | 25.5 |

[Table 3]

| Synthesis Example | Mw | Mw/Mn | Copolymerization ratio (a1):(a2) or (a1):(a2') | Fluorine content (mass%) |
|---|---|---|---|---|
| 1 | 36,000 | 2.38 | 30:70 | 32.3 |
| 2 | 15,000 | 1.88 | 30:70 | 32.3 |
| 3 | 66,000 | 2.45 | 30:70 | 32.3 |
| 4 | 106,000 | 2.44 | 30:70 | 32.3 |
| 5 | 43,000 | 2.20 | 47:53 | 41.7 |
| 6 | 46,000 | 2.07 | 67:33 | 49.2 |
| 7 | 50,000 | 1.97 | 89:11 | 54.9 |
| 8 | 48,000 | 2.14 | 51:49 | 39.8 |
| 9 | 48,000 | 2.09 | 51:49 | 40.5 |
| 10 | 45,000 | 2.65 | 43:57 | 31.8 |
| 11 | 30,000 | 1.77 | 32:68 | 34.9 |
| 12 | 27,000 | 2.00 | 49:51 | 43.5 |
| 13 | 35,000 | 1.83 | 32:68 | 24.5 |
| 14 | 30,000 | 1.93 | 49:51 | 34.6 |
| 17 | 22,000 | 2.13 | 29:71 | 31.4 |
| 18 | 23,000 | 2.13 | 29:71 | 31.8 |
| 19 | 16,000 | 2.01 | 100:0 | 57.2 |
| 20 | 12,000 | 2.15 | 0:100 | 0 |

[Table 4]

| Synthesis Example | Mw | Mw/Mn | Copolymerization ratio (a1):(a2):(a3) | Florine content (mass%) |
|---|---|---|---|---|
| 15 | 41,000 | 1.54 | 36:19:45 | 21.9 |
| 16 | 57,000 | 4.30 | 31:16:53 | 24.4 |

(Example 1)

10.0 g of the polymer (Synthesis Example 1), 1.0 g of CPI210S (absorption coefficient: 98 mL·g$^{-1}$·cm$^{-1}$) as a photoinitiator and 20.4 g of PGMEAas a solvent were put in a glass vial (50 mL) and thoroughly stirred to obtain a uniform solution. The obtained solution was filtered through a PTFE filter having a pore size of 0.20 μm to prepare a photosensitive composition. By using the photosensitive composition, the resolution, the adhesion, the fluorescence intensity and the liquid repellency were evaluated. The evaluation methods will be described later.

(Examples 2 to 5 and 10)

Also in each of Examples 2 to 5 and Example 10, a photosensitive composition was prepared in the same manner as in Example 1 except that the polymer was changed to the polymer synthesized in each of Synthesis Example 2 to 5 and Synthesis Example 10, and by using the photosensitive composition, the resolution, the adhesion, the fluorescence intensity and the liquid repellency were evaluated.

(Example 6)

4.0 g of the polymer (Synthesis Example 6), 0.4 g of CPI210S (absorption coefficient: 98 mL·g$^{-1}$·cm$^{-1}$) as a photoinitiator and 15.6 g of OFPO as a solvent were put in a glass vial (30 mL) and thoroughly stirred to obtain a uniform solution. The obtained solution was filtered through a PTFE filter having a pore size of 0.20 $\mu$m to prepare a photosensitive composition. By using the photosensitive composition, the resolution, the adhesion, the fluorescence intensity and the liquid repellency were evaluated.

(Examples 7 to 9)

Also in each of Examples 7 to 9, a photosensitive composition was prepared in the same manner as in Example 6 except that the polymer was changed to the polymer synthesized in each of Synthesis Examples 7 to 9, and by using the photosensitive composition, the resolution, the adhesion, the fluorescence intensity and the liquid repellency were evaluated.

(Example 11)

Also in Example 11, a photosensitive composition was prepared in the same manner as in Example 1 except that the photoinitiator was changed to WPAG199 (absorption coefficient: 151 mL·g$^{-1}$·cm$^{-1}$), and by using the photosensitive composition, the resolution, the adhesion, the fluorescence intensity and the liquid repellency were evaluated.

(Example 12)

10.0 g of the polymer (Synthesis Example 13), 1.0 g of IR184 (absorption coefficient: 89 mL·g$^{-1}$·cm$^{-1}$) as a photoinitiator and 20.4 g of PGMEA as a solvent were put in a glass vial (50 mL) and thoroughly stirred to obtain a uniform solution. The obtained solution was filtered through a PTFE filter having a pore size of 0.20 $\mu$m to prepare a photosensitive composition. By using the photosensitive composition, the resolution, the adhesion, the fluorescence intensity and the liquid repellency were evaluated.

(Example 13)

Also in Example 13, a photosensitive composition was prepared in the same manner as in Example 12 except that the photoinitiator was changed to IR127 (absorption coefficient: 107 mL·g$^{-1}$·cm$^{-1}$), and by using the photosensitive composition, the resolution, the adhesion, the fluorescence intensity and the liquid repellency were evaluated.

(Example 14)

Also in Example 14, a photosensitive composition was prepared in the same manner as in Example 12 except that the polymer was changed to the polymer synthesized in Synthesis Example 14, and by using the photosensitive composition, the resolution, the adhesion, the fluorescence intensity and the liquid repellency were evaluated.

(Example 15)

Also in Example 15, a photosensitive composition was prepared in the same manner as in Example 1 except that the polymer was changed to the polymer synthesized in Synthesis Example 15, the solvent was changed to MEK and the development was carried out with PGMEA, and by using the photosensitive composition, the resolution, the adhesion, the fluorescence intensity, the adhesion of a protein, the adhesion of cells and the liquid repellency were measured.

(Example 16)

Also in Example 16, a photosensitive composition was prepared in the same manner as in Example 1 except that the polymer was changed to the polymer synthesized in Synthesis Example 16, the solvent was changed to OFPO, and the development was carried out with isopropanol, and by using the photosensitive composition, the resolution, the adhesion, the fluorescence intensity, the adhesion of a protein, the adhesion of cells and the liquid repellency were evaluated.

(Example 17)

The reaction liquid in Synthesis Example 17 was filtered through a PTFE filter having a pore size of 0.2 $\mu$m, and 4.17 g of the filtered reaction liquid and 0.13 g of CPI210S (absorption coefficient: 98 mL·g$^{-1}$·cm$^{-1}$) as a photoinitiator were put in a glass vials (6 mL) and thoroughly stirred to prepare a photosensitive composition. By using the photosensitive composition, the resolution, the adhesion, the fluorescence intensity and the liquid repellency were evaluated.

(Example 18)

A photosensitive composition was prepared in the same manner as in Example 17 except that the reaction liquid was changed to the reaction liquid in Synthesis Example 18, and by using the photosensitive composition, the resolution, the adhesion, the fluorescence intensity and the liquid repellency were evaluated.

(Example 19)

A photosensitive composition was prepared in the same manner as in Example 6 except that the reaction liquid was changed to one having the reaction liquids in Synthesis Example 19 and Synthesis Example 20 mixed in amounts of 3.0 g and 7.0 g, respectively, and by using the photosensitive composition, the resolution, the adhesion, the fluorescence intensity and the liquid repellency were evaluated.

(Example 20)

A photosensitive composition was prepared in the same manner as in Example 6 except that the reaction liquid was changed to one having the reaction liquids in Synthesis Example 19 and Synthesis Example 20 mixed in amounts of 5.0 g and 5.0 g, respectively, and by using the photosensitive composition, the resolution, the adhesion, the fluorescence intensity and the liquid repellency were evaluated.

(Example 21)

A photosensitive composition was prepared in the same manner as in Example 6 except that the reaction liquid was changed to one having the reaction liquids in Synthesis Example 19 and Synthesis Example 20 mixed in amounts of 7.0 g and 3.0 g, respectively, and by using the photosensitive composition, the resolution, the adhesion, the fluorescence intensity and the liquid repellency were evaluated.

(Example 22)

A photosensitive composition was prepared in the same manner as in Example 6 except that the reaction liquid was changed to one having the reaction liquids in Synthesis Example 19 and Synthesis Example 20 mixed in amounts of 9.0 g and 1.0 g, respectively, and by using the photosensitive composition, the resolution, the adhesion, the fluorescence intensity and the liquid repellency were evaluated.

(Comparative Example 1)

Also in Comparative Example 1, a photosensitive composition was prepared in the same manner as in Example 1 except that the photoinitiator was changed to IRPAG103 (absorption coefficient: 11,000 mL·g$^{-1}$·cm$^{-1}$), and by using the photosensitive composition, the resolution, the adhesion, the fluorescence intensity and the liquid repellency were evaluated.

(Comparative Example 2)

Also in Comparative Example 2, a photosensitive composition was prepared in the same manner as in Example 1 except that the photoinitiator was changed to CPI310B (absorption coefficient: 600 mL·g$^{-1}$·cm$^{-1}$), and by using the photosensitive composition, the resolution, the adhesion, the fluorescence intensity and the liquid repellency were evaluated.

(Comparative Example 3)

Also in Comparative Example 3, a photosensitive composition was prepared in the same manner as in Example 1 except that the photoinitiator was changed to CPI410S (absorption coefficient: 4,300 mL·g$^{-1}$·cm$^{-1}$), and by using the photosensitive composition, the resolution, the adhesion, the fluorescence intensity and the liquid repellency were evaluated.

(Comparative Example 4)

Also in Comparative Example 4, a photosensitive composition was prepared in the same manner as in Example 12 except that the photoinitiator was changed to IR907 (absorption coefficient: 467 mL·g$^{-1}$·cm$^{-1}$), and by using the photosensitive composition, the resolution, the adhesion, the fluorescence intensity and the liquid repellency were evaluated.

(Comparative Example 5)

Also in Comparative Example 5, a photosensitive composition was prepared in the same manner as in Example 12 except that the photoinitiator was changed to IR819 (absorption coefficient: 2,309 mL·g$^{-1}$·cm$^{-1}$), and by using the photosensitive composition, the resolution, the adhesion, the fluorescence intensity and the liquid repellency were evaluated.

(Comparative Example 6)

Also in Comparative Example 6, a photosensitive composition was prepared in the same manner as in Example 12 except that the photoinitiator was changed to OXE01 (absorption coefficient: 6,969 mL·g$^{-1}$·cm$^{-1}$), and by using the photosensitive composition, the resolution, the adhesion, the fluorescence intensity and the liquid repellency were evaluated.

[Evaluation methods]

(Resolution)

On a 4-inch quartz glass substrate, a photosensitive composition was spin-coated at 2,000 rpm/min. for 30 seconds and heated at 100°C for 120 seconds by using a hot plate, to form a dried coating film. Using a high-pressure mercury lamp as the light source, the dried coating film was exposed via a mask (a mask capable of forming a pattern of circular holes having diameters of 15 $\mu$m and 20 $\mu$m) so that the exposure energy would be 400 mJ/cm$^2$, to cure portions (exposed portions) of the dried coating film. In order to accelerate curing of the dried coating film, the film was heated at 120°C for 120 seconds by using a hot plate. With respect to each of Examples 17 and 18, the dried coating film was exposed so that the exposure energy would be 1,000 mJ/cm$^2$, to cure portions (exposed portions) of the dried coating film.

To the cured film, dipping development was carried out for 60 seconds by using, as the developer (solvent), the same solvent as the solvent in the photosensitive composition, to form a cured film having a pattern of holes corresponding to the mask. The thickness of the cured film at the portions which had not been removed by development was 3.0 $\mu$m.

The cured film was observed by a microscope (manufactured by KEYENCE Corporation, VHX DIGITAL MICROSCOPE), whereby a resin composition having 15 $\mu$m holes opened, was judged to be good ($\bigcirc$) in resolution. One having no 15 $\mu$m holes opened, but having 20 $\mu$m holes opened, was judged to be usual ($\Delta$), and one having no 20 $\mu$m holes opened, or having no pattern remained, was judged to be defective ($\times$).

(Adhesion)

On a 4-inch quartz glass substrate, a photosensitive composition was spin-coated at 2,000 rpm/min. for 30 seconds and heated at 100°C for 120 seconds by using a hot plate, to form a dried coating film. Using a high-pressure mercury lamp as the light source, the dried coating film was exposed so that the exposure energy would be 400 mJ/cm$^2$, to cure the dried coating film. In order to accelerate curing of the dried coating film, by using a hot plate, the film was heated at 120°C for 120 seconds, followed by curing at 200°C for 30 minutes. The cured film was subjected to a cross-cut peeling test (JIS5600-5-6). One having no peeling observed was judged to be such that the adhesion is good (○). One having peeling partially observed, was judged to be usual (△), and one having entirely peeled was judged to be defective (×).

(Fluorescence intensity and liquid repellency)

On a 25 mm × 50 mm quartz glass substrate, a photosensitive composition was spin-coated, and by using a hot plate, heated at 100°C for 120 seconds to form a dried coating film having a thickness of 0.8 μm. By using a high-pressure mercury lamp as a light source, the dried coating film was exposed so that the exposure energy would be 1,000 mJ/cm$^2$ and thus cured. In order to accelerate curing of the dried coating film, by using a hot plate, the film was heated at 120°C for 120 seconds, followed by curing at 200°C for 30 minutes.

The fluorescence intensity of the cured film was measured by a microarray scanner (manufactured by Molecular Devices, GenePix 4000B) (excitation wavelength: 532 nm, resolution: 5 μm, laser output: 100%, voltage of photomultiplier: 1,000 V). One with the fluorescence intensity being at most 15,000, was judged to be such that the fluorescence intensity is good. The fluorescence intensity is more preferably at most 10,000, further preferably at most 5,000, particularly preferably at most 4,000.

At the surface coated with a resin composition for a cured film, the contact angles of water and PGMEA were measured by a full automatic contact angle meter (manufactured by Kyowa Interface Science Co., Ltd., DM-701). The liquid amount was 2.0 μL, and the contact angle after retention for 2,000 ms after dropping, was measured. One with the water contact angle being at least 100 degrees, or with the contact angle of PGMEA being at least 45 degrees, was judged to be such that the liquid repellency is good. The results are shown in Table 5.

(Protein adhesion)

The protein adsorption rate Q was obtained by the following procedure (1) to (6).

(1) Preparation of wells:

The photosensitive composition obtained in Example 6, 15 or 16 was dispensed in an amount of 2.2 mL in each of 3 wells of a microplate (manufactured by AGC TECHNO GLASS CO., LTD.) with 24 wells, and left to stand for one day to evaporate the solvent, and the dried coating film was exposed so that the exposure energy became 400 mJ/cm$^2$ to form a coating layer (liquid-repellent film) on the well surface.

(2) Preparation of a coloring liquid and a protein solution:

As a coloring liquid, one obtained by mixing 50 mL of a peroxidase coloring liquid (3,3',5,5'-tetramethylbenzidine (TMBZ), manufactured by KPL Co.)) and 50 mL of TMB Peroxidase Substrate (manufactured by KPL Co.), was used. Further, as a protein solution, one obtained by diluting a protein (POD-goat anti mouse IgG, manufactured by Biorad) to 16,000-fold with a phosphate buffer solution (D-PBS, manufactured by Sigma), was used.

(3) Adsorption of protein:

In the microplate with 24 wells having the coating layer formed in the above (1), in each of the wells having the coating layer formed thereon, 2 mL of the above protein solution was dispensed (2 mL per every one well) and left to stand at room temperature for 1 hour. As a blank, in a microplate with 96 wells uncoated, in each of 3 wells, 2 μL of the above protein solution was dispensed.

(4) Washing of wells:

Then, the above microplate with 24 wells was washed four times with 4 mL of a phosphate buffer solution (D-PBS (Dulbecco's phosphate buffered saline), manufactured by Sigma) having a surfactant (Tween20, manufactured by Wako Pure Chemical Industries, Ltd.) incorporated in an amount of 0.05 mass% (4 mL used per every one well).

(5) Dispensing of coloring liquid:

Then, to the microplate with 24 wells after the washing, 2 mL of the above coloring liquid was dispensed (2 mL used per every one well), and a coloring reaction was carried out for 7 minutes, whereupon 1 mL of 2N sulfuric acid was added (1 mL used per every one well) to terminate the coloring reaction. As a blank, to each well of the microplate with 96 wells, 100 $\mu$L of the coloring liquid was dispensed (100 $\mu$L used per every one well), and a coloring reaction was carried out for 7 minutes, whereupon 50 $\mu$L of 2N sulfuric acid was added (50 $\mu$L used per every one well) to terminate the coloring reaction.

(6) Measurement of absorbance and calculation of protein adsorption rate Q:

Then, from each well of the microplate with 24 wells, 150 $\mu$L of liquid was taken and transferred, respectively, to 3 wells of the microplate with 96 wells, whereupon the absorbance at a wavelength of 450 nm was measured by MTP-810Lab (manufactured by Corona Electric Co., Ltd.). Here, the average value of absorbance (N=3) of the blank was deemed to be $A_0$. The absorbance of the liquid transferred from the microplate with 24 wells to the microplate with 96 wells was deemed to be $A_1$, and the protein adsorption rate $Q_1$ was obtained by the following formula. The average value of three protein adsorption rates $Q_1$ was taken as the protein adsorption rate Q. The results are shown in Table 6. The protein adsorption rate is preferably at most 5%, particularly preferably at most 1%.

$$Q_1 = A_1/\{A_0 \times (100/\text{dispense volume of the protein solution of blank})\} \times 100 = A_1/\{A_0 \times (100/2\ \mu L)\} \times 100\ [\%]$$

(Cell adhesion)

On a 27 mm$\varphi$ glass substrate, a photosensitive composition was spin-coated at 2,000 rpm for 30 seconds, and by using a hot plate, heated at 100°C for 120 seconds to form a dried coating film. Using a high-pressure mercury lamp as the light source, the dried coating film was exposed via a mask (a mask capable of forming a pattern of circular holes having a diameter of 500 $\mu$m), so that the exposure energy would be 400 mJ/cm$^2$, to cure portions (exposed portions) of the dried coating film. In order to accelerate curing of the dried coating film, the film was heated at 120°C for 120 seconds by using a hot plate.

To the cured film, dipping development was carried out for 60 seconds by using the above-mentioned solvent as the developer to form a cured film (liquid-repellent surface) having a pattern of holes corresponding to the mask. The thickness of the cured film at the portions not removed by the development was 3.0 $\mu$m. On the other hand, at the portions removed by the development, the glass surface was exposed.

2 mL of a suspension of 800,000 TIG-3 cells was seeded on the cured film having the pattern of holes and cultured at 37°C for 9 hours in a 5% carbon dioxide atmosphere. Observation was made by using an optical microscope, and the cell adhesion rate (%) was calculated by the following formula (I) from the number of cells at the glass surface of 500 $\mu$m$\varphi$ and the number of cells at the liquid-repellent surface of 500 $\mu$m$\varphi$, whereupon one having the calculated value being at most 40% was judged to be good.

Cell adhesion rate (%) = (number of cells at liquid-repellent surface)/(number of cells at glass surface + number of cells at liquid-repellent surface) $\times$ 100 (I)

The results of evaluations carried out in Examples 1 to18 and Comparative Examples 1 to 6 are shown in Tables 5 and 6.

[Table 5]

| | Polymer | Photoinitiator | | Solvent | Water contact angle | PGMEA contact angle | Fluorescence intensity | Resolution | Adhesion |
|---|---|---|---|---|---|---|---|---|---|
| | Synthesis Example | Type | Absorption coefficient | | | | | | |
| Example 1 | 1 | CPI210S | 98 | PGMEA | 106 | 53 | 925 | ○ | ○ |
| Example 2 | 2 | CPI210S | 98 | PGMEA | 105 | 53 | 1335 | ○ | ○ |
| Example 3 | 3 | CPI210S | 98 | PGMEA | 105 | 53 | 2460 | ○ | ○ |
| Example 4 | 4 | CPI210S | 98 | PGMEA | 106 | 53 | 1784 | ○ | ○ |
| Example 5 | 5 | CPI210S | 98 | PGMEA | 109 | 54 | 2050 | ○ | ○ |
| Example 6 | 6 | CPI210S | 98 | OFPO | 112 | 57 | 1340 | ○ | ○ |
| Example 7 | 7 | CPI210S | 98 | OFPO | 114 | 55 | 1731 | ○ | ○ |
| Example 8 | 8 | CPI210S | 98 | OFPO | 106 | 53 | 2355 | ○ | ○ |
| Example 9 | 9 | CPI210S | 98 | OFPO | 108 | 52 | 2519 | ○ | ○ |
| Example 10 | 10 | CPI210S | 98 | PGMEA | 104 | 47 | 1164 | ○ | ○ |
| Example 11 | 1 | WPAG199 | 151 | PGMEA | 105 | 53 | 4486 | ○ | ○ |
| Example 12 | 13 | IR184 | 89 | PGMEA | 106 | 56 | 2278 | ○ | ○ |
| Example 13 | 13 | IR127 | 107 | PGMEA | 106 | 56 | 14163 | ○ | ○ |
| Example 14 | 14 | IR184 | 89 | PGMEA | 108 | 57 | 3542 | ○ | ○ |
| Example 15 | 15 | CPI210S | 98 | MEK | 110 | 65 | 200 | ○ | ○ |
| Example 16 | 16 | CPI210S | 98 | OFPO | 123 | 61 | 2030 | ○ | ○ |
| Example 17 | 17 | CPI210S | 98 | PGMEA | 107 | 56 | 12000 | ○ | ○ |
| Example 18 | 18 | CPI210S | 98 | PGMEA | 107 | 57 | 6500 | ○ | ○ |
| Example 19 | 19/20=3/7 | CPI210S | 98 | OFPO | 105 | 53 | 1250 | ○ | ○ |
| Example 20 | 19/20=5/5 | CPI210S | 98 | OFPO | 106 | 53 | 1830 | ○ | ○ |
| Example 21 | 19/20=7/3 | CPI210S | 98 | OFPO | 109 | 56 | 1590 | ○ | ○ |
| Example 22 | 19/20=9/1 | CPI210S | 98 | OFPO | 112 | 57 | 1650 | ○ | ○ |
| Comparative Example 1 | 1 | IRPAG103 | 11000 | PGMEA | 105 | 53 | 65535 | ○ | ○ |

EP 3 376 286 A1

(continued)

| | Polymer | Photoinitiator | | Solvent | Water contact angle | PGMEA contact angle | Fluorescence intensity | Resolution | Adhesion |
|---|---|---|---|---|---|---|---|---|---|
| | Synthesis Example | Type | Absorption coefficient | | | | | | |
| Comparative Example 2 | 1 | CPI310B | 600 | PGMEA | 105 | 53 | 60066 | ○ | ○ |
| Comparative Example 3 | 1 | CPI410S | 4300 | PGMEA | 105 | 53 | 65511 | ○ | ○ |
| Comparative Example 4 | 13 | IR907 | 467 | PGMEA | 106 | 56 | 65352 | ○ | ○ |
| Comparative Example 5 | 13 | IR819 | 2309 | PGMEA | 106 | 56 | 61033 | ○ | ○ |
| comparative Example 6 | 13 | OXE01 | 6969 | PGMEA | 106 | 55 | 65213 | ○ | ○ |

[Table 6]

| | Polymer | Photoinitiator | | Solvent | Protein adsorption rate Q (%) | Cell adhesion rate (%) |
|---|---|---|---|---|---|---|
| | Synthesis Example | Type | Absorption coefficient | | | |
| Example 6 | 6 | CPI210S | 98 | OFPO | 1.6 | 17 |
| Example 15 | 15 | CPI210S | 98 | MEK | 0.02 | 33 |
| Example 16 | 16 | CPI210S | 98 | OFPO | 0.4 | - |

In order to obtain a cured film having a low fluorescence intensity, a photoinitiator having an absorption coefficient at 365 nm of at most 400 [mL·g$^{-1}$·cm$^{-1}$] may be used.

INDUSTRIAL APPLICABILITY

According to the present invention, it is possible to obtain a biochip whereby light which becomes noise at the time of fluorescence analysis is suppressed.

The entire disclosures of Japanese Patent Application No. 2015-220176 filed on November 10, 2015 and Japanese Patent Application No. 2016-136415 filed on July 8, 2016 including specifications, claims and summaries are incorporated herein by reference in their entireties.

**Claims**

1. A photosensitive composition which comprises the following polymer (A), or the following polymer (B) and the following polymer (C), and further a photoinitiator having an absorption coefficient at a wavelength of 365 nm of at most 400 [mL·g$^{-1}$·cm$^{-1}$], and which is to be used to form a liquid repellent film on a liquid contact surface of a biochip for fluorescence analysis,
Polymer (A): a polymer having a polymerizable crosslinkable group and a fluoroalkyl group which may have an etheric oxygen atom between carbon atoms,
Polymer (B): a polymer other than the polymer (A), having a fluoroalkyl group which may have an etheric oxygen atom between carbon atoms,
Polymer (C): a polymer other than the polymer (A), having a polymerizable crosslinkable group.

2. The photosensitive composition according to Claim 1, wherein the fluoroalkyl group in the polymer (A) and the polymer (B) is a fluoroalkyl group having a perfluoroalkyl moiety having 4, 5 or 6 carbon atoms.

3. The photosensitive composition according to Claim 1, wherein the fluoroalkyl group in the polymer (A) and the polymer (B) is a fluoroalkyl group having a perfluoroalkyl moiety having 4 to 8 carbon atoms and having from 1 to 3 etheric oxygen atoms between carbon atoms.

4. The photosensitive composition according to any one of Claims 1 to 3, wherein the polymerizable crosslinkable group in the polymer (A) and the polymer (C) is a group having an ethylenic double bond, a group having a three-membered cyclic ether structure, or a group having a four-membered cyclic ether structure.

5. The photosensitive composition according to any one of Claims 1 to 4, wherein the weight average molecular weight of each of the polymer (A), the polymer (B) and the polymer (C) is from 5,000 to 500,000.

6. The photosensitive composition according to any one of Claims 1 to 5, wherein the photosensitive composition is a photosensitive composition comprising the polymer (A).

7. The photosensitive composition according to Claim 6, wherein the polymer (A) is a polymer comprising a structural unit having a fluoroalkyl group which may have an etheric oxygen atom between carbon atoms, a structural unit having a polymerizable crosslinkable group, and optionally a structural unit other than the above units.

8. The photosensitive composition according to Claim 7, wherein the content proportions of the respective structural units in the polymer (A) are such that, based on all structural units, the structural unit having a fluoroalkyl group is from 20 to 95 mol%, the structural unit having a polymerizable crosslinkable group is from 5 to 80 mol%, and the structural unit other than the above units is from 0 to 60 mol%.

9. The photosensitive composition according to any one of Claims 1 to 8, wherein the photoinitiator is a photo-radical generator or a photo-acid generator.

10. The photosensitive composition according to any one of Claims 1 to 9, wherein the photosensitive composition further contains a solvent.

11. A method for producing a biochip for fluorescence analysis, **characterized by** applying the photosensitive composition as defined in any one of Claims 1 to 10 on a liquid contact surface of a substrate of the biochip for fluorescence analysis, and, when the coating film of the photosensitive composition has a solvent, removing said solvent, followed by exposure and development, to form a liquid repellent film having through-holes formed in the thickness direction.

12. The method for producing a biochip for fluorescence analysis according to Claim 11, wherein the substrate is a substrate having a lyophilic surface, and the liquid repellent film is formed on the lyophilic surface of the substrate.

13. The method for producing a biochip for fluorescence analysis according to Claim 11 or 12, wherein the fluorine content in the liquid repellent film formed is from 15 to 75 mass%.

14. A biochip for fluorescence analysis comprising a substrate and a liquid repellent film provided on a liquid contact surface of the substrate and having through-holes formed in the thickness direction, wherein
the liquid repellent film has a fluorescence intensity of at most 15,000 as measured by the following measurement method, a water contact angle of at least 100 degrees, and a protein adsorption rate Q of at most 5% as measured by the following measurement method,
(Measurement method for fluorescence intensity)
The above liquid repellent film is formed on a quartz glass substrate in a thickness of 0.8 $\mu$m, and the fluorescence intensity of the liquid repellent film is measured by a microarray scanner (manufactured by Molecular Devices, GenePix 4000B), under conditions of an excitation wavelength of 532 nm, a laser power of 100% and a photomultiplier voltage of 1,000 V,
(Measurement method for protein adsorption rate Q)
The protein adsorption rate Q is obtained by the following procedure (1) to (6):

(1) Preparation of wells: In each of 3 wells of a microplate with 24 wells, the above liquid-repellent film is formed on the well surface to cover said well surface.
(2) Preparation of a coloring liquid and a protein solution: As a coloring liquid, one obtained by mixing 50 mL of a peroxidase coloring liquid (3,3',5,5'-tetramethylbenzidine) and 50 mL of 3,3',5,5'-tetramethylbenzidine peroxidase substrate, is used, and, as a protein solution, one obtained by diluting a protein (POD-goat anti mouse IgG, manufactured by Biorad) to 16,000-fold with a phosphate buffer solution (D-PBS, manufactured by Sigma), is used.
(3) Adsorption of protein: In the microplate with 24wells having the liquid repellent film formed in the above (1), in each of the wells having the liquid repellent film formed thereon, 2mL of the protein solution is dispensed and left to stand at room temperature for 1 hour. As a blank, in a microplate with 96 wells uncoated, in each of 3 wells, 2$\mu$L of the protein solution is dispensed.
(4) Washing of wells: The microplate with 24 wells subjected to adsorption of the protein in the above (3) is washed four times with 4 mL of a phosphate buffer solution (D-PBS, manufactured by Sigma) having a surfactant (Tween20, manufactured by Wako Pure Chemical Industries, Ltd.) incorporated in an amount of 0.05 mass%.
(5) Dispensing of coloring liquid: To each well of the microplate with 24 wells after the washing in the above (4), 2 mL of the above coloring liquid is dispensed, and a coloring reaction is carried out for 7 minutes, whereupon 1 mL of 2N sulfuric acid is added to terminate the coloring reaction. As a blank, to each well of the microplate with 96 wells, 100 $\mu$L of the coloring liquid is dispensed, and a coloring reaction is carried out for 7 minutes, whereupon 50 $\mu$L of 2N sulfuric acid is added to terminate the coloring reaction.
(6) Measurement of absorbance and calculation of protein adsorption rate Q:

From each well of the microplate with 24 wells, 150 $\mu$L of liquid is taken and transferred, respectively, to 3 wells of the microplate with 96 wells, whereupon the absorbance at a wavelength of 450 nm is measured by MTP-810Lab (manufactured by Corona Electric Co., Ltd.). Here, the average value of absorbance of the blank is deemed to be $A_0$.

The absorbance of the liquid transferred from the microplate with 24 wells to the microplate with 96 wells is deemed to be $A_1$, and the protein adsorption rate $Q_1$ is obtained by the following formula. The average value of three protein adsorption rates $Q_1$ is taken as the protein adsorption rate Q.

$$Q_1 = A_1/\{A_0 \times (100/\text{dispense volume of the protein solution of blank})\} \times 100 =$$

$$A_1/\{A_0 \times (100/2\ \mu L)\} \times 100\ [\%]$$

**15.** The biochip for fluorescence analysis according to Claim 14, wherein the fluorine content in the liquid repellent film is from 15 to 75 mass%.

## INTERNATIONAL SEARCH REPORT

| International application No. |
| --- |
| PCT/JP2016/083268 |

### A. CLASSIFICATION OF SUBJECT MATTER
*G03F7/038*(2006.01)i, *G01N37/00*(2006.01)n

According to International Patent Classification (IPC) or to both national classification and IPC

### B. FIELDS SEARCHED

Minimum documentation searched (classification system followed by classification symbols)
G03F7/038

Documentation searched other than minimum documentation to the extent that such documents are included in the fields searched

| | | | |
| --- | --- | --- | --- |
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X<br>A | JP 2008-287251 A (Daikin Industries, Ltd.), 27 November 2008 (27.11.2008), claims; paragraphs [0119] to [0139]; tables 1, 3; examples 21 to 26; comparative examples 2, 4, 6, 8 & KR 10-2008-0093929 A & TW 200916956 A | 1-2,4-10<br>3,11-15 |
| X<br>A | JP 2012-073603 A (Sumitomo Chemical Co., Ltd.), 12 April 2012 (12.04.2012), claims; paragraphs [0175] to [0179]; table 3, photosensitive resin composition 5 & CN 102385248 A & KR 10-2012-0022670 A & TW 201224654 A | 1-2,4-10<br>3,11-15 |

☒ Further documents are listed in the continuation of Box C. ☐ See patent family annex.

| | | |
| --- | --- | --- |
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
| --- | --- |
| 06 December 2016 (06.12.16) | 20 December 2016 (20.12.16) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
| --- | --- |

Form PCT/ISA/210 (second sheet) (January 2015)

**INTERNATIONAL SEARCH REPORT**

| International application No. |
|---|
| PCT/JP2016/083268 |

C (Continuation).  DOCUMENTS CONSIDERED TO BE RELEVANT

| Category* | Citation of document, with indication, where appropriate, of the relevant passages | Relevant to claim No. |
|---|---|---|
| X<br>A | JP 2012-108499 A  (Sumitomo Chemical Co., Ltd.),<br>07 June 2012 (07.06.2012),<br>claims; paragraphs [0197], [0205] to [0207];<br>table 3, photosensitive resin composition 3;<br>comparative examples<br>& WO 2012/057058 A1      & TW 201229670 A<br>& CN 103189797 A         & KR 10-2014-0007799 A | 1-2,4-10<br>3,11-15 |
| X<br>A | WO 2015/163379 A1  (Asahi Glass Co., Ltd.),<br>29 October 2015 (29.10.2015),<br>claims; paragraphs [0164], [0172] to [0175],<br>[0186]; tables 1, 2; synthesis example 4;<br>example 4<br>& TW 201546546 A | 1-2,4-7,9-10<br>3,8,11-15 |
| A | JP 2009-075261 A  (Nippon Kayaku Co., Ltd.),<br>09 April 2009 (09.04.2009),<br>claims; paragraphs [0002], [0003]; examples<br>(Family: none) | 1-15 |

Form PCT/ISA/210 (continuation of second sheet) (January 2015)

## INTERNATIONAL SEARCH REPORT

| International application No. |
|---|
| PCT/JP2016/083268 |

---

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:
        See extra sheet.

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

International application No.

PCT/JP2016/083268

Continuation of Box No.III of continuation of first sheet(2)

(Invention 1) claims 1-13
Documents 1-4 disclose photosensitive resin compositions which comprise polymer (A) and further contain a photoinitiator having an absorption coefficient at a wavelength of 365 nm of 400 $[mL \cdot g^{-1} \cdot cm^{-1}]$ or less. Thus, claim 1 and claims 2 and 4-10 depending on claim 1 lack novelty in the light of documents 1-4 and, therefore, have no special technical feature.
Consequently, claims 1, 2 and 4-10 are classified into Invention 1.
Further, claims 3 and 11-13 are classified into Invention 1, since these claims are dependent on claim 1.
(Invention 2) claims 14-15
Claim 14 and claim 15 depending on claim 14 have a technical feature common to claim 1 that is identified as Invention 1, said technical feature being a biochip for fluorescence analysis provided with a liquid-repellent film.
However, the above-said technical feature cannot be considered to be a special technical feature, since the technical feature does not make a contribution over the prior art in the light of the contents set forth in the description of this application.
Further, there is no other same or corresponding special technical feature between these inventions.
In addition, claims 14-15 have no relationship such that these claims are substantially same as or equivalent to any claim classified into Invention 1.
Consequently, claims 14-15 cannot be classified into Invention 1.
Document 1: JP 2008-287251 A (Daikin Industries, Ltd.), 27 November 2008 (27.11.2008), claims; paragraphs [0119] to [0139]; tables 1, 3; examples 21 to 26; comparative examples 2, 4, 6, 8 & KR 10-2008-0093929 A & TW 200916956 A
Document 2: JP 2012-073603 A (Sumitomo Chemical Co., Ltd.), 12 April 2012 (12.04.2012), claims; paragraphs [0175] to [0179]; table 3; photosensitive resin composition 5 & CN 102385248 A & KR 10-2012-0022670 A & TW 201224654 A
Document 3: JP 2012-108499 A (Sumitomo Chemical Co., Ltd.), 07 June 2012 (07.06.2012), claims; paragraphs [0197], [0205] to [0207]; table 3; photosensitive resin composition 3; comparative examples & WO 2012/057058 A1 & TW 201229670 A & CN 103189797 A & KR 10-2014-0007799 A
Document 4: WO 2015/163379 A1 (Asahi Glass Co., Ltd.), 29 October 2015 (29.10.2015), claims; paragraphs [0164], [0172] to [0175], [0186]; tables 1, 2; synthesis example 4; example 4 & TW 201546546 A


Claim 14 and claim 15 depending on claim 14 include any biochips for fluorescence analysis provided with a liquid-repellent film which has such properties that the fluorescence intensity is 15,000 or less, the contact angle with water is 100° or more and the protein adsorptivity is 5% or less.

(Continued to next extra sheet)

Form PCT/ISA/210 (extra sheet) (January 2015)

**INTERNATIONAL SEARCH REPORT**

| International application No. |
|---|
| PCT/JP2016/083268 |

In the description, however, it is recognized that only biochips for fluorescence analysis provided with a liquid-repellent film formed of a photosensitive composition which comprises polymer (A) or polymers (B) and (C) and further contains a photoinitiator having an absorption coefficient at a wavelength of 365 nm of 400 [mL·g$^{-1}$·cm$^{-1}$] or less are exclusively disclosed under the provision of Article 5 of the PCT. Therefore, these claims are not supported under the provision of Article 6 of the PCT.

Such being the case, the search was made exclusively on biochips for fluorescence analysis provided with a liquid-repellent film formed of such a photosensitive composition as specified above.

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012121310 A
- WO 2006129800 A
- JP 2009075261 A

- JP 2004359616 A
- JP 2015220176 A
- JP 2016136415 A